# EUROPEAN PATENT APPLICATION

(11) **EP 1 816 199 A1**
(43) Date of publication of application: **08.08.2007**
(21) Application number: 07004548.9
(22) Date of filing: 22.09.1998
(51) Int. Cl.: C12N 15/16, C07K 14/59, A61K 38/24, G01N 33/68

(54) **Mutants of thyroid stimulating hormone and methods based thereon**

(30) Priority: 22.09.1997 US 939472
(62) Divisional of application: 98948422.5
(71) Applicant: University of Maryland, Baltimore, Baltimore, MD 21201-1602 (US)
(72) Inventor: Weintraub, Bruce D, Rockville, MD 20852 (US); Szkudlinski, Mariusz W, Potomac, MD 20854 (US)
(74) Representative: Khoo, Chong-Yee

(57) **Abstract**

The present invention is based upon the discovery that mutant α subunits and mutant β subunits each comprising amino acid substitutions relative to the wild type can be produced and assembled to form a mutant TSH heterodimer or TSH analog that possesses higher bioactivity *in vitro* and longer half life *in vivo.* Accordingly, the present invention provides methods for using mutant TSH heterodimers, TSH analogs, fragments, and derivatives thereof for treating or preventing diseases of the thyroid, in particular thyroid cancer. The invention also relates to methods of diagnosis, prognosis and monitoring for thyroid-related functions. Pharmaceutical and diagnostic compositions, methods of using mutant TSH heterodimers and TSH analogs with utility for treatment and prevention of metabolic and reproductive diseases are also provided.

## Description

### 1. FIELD OF THE INVENTION

The present invention relates to mutants of thyroid stimulating hormone, and derivatives and analogs thereof. Methods for producing thyroid stimulating hormone mutants, derivatives and analogs are also provided. The invention further relates to pharmaceutical compositions and methods of diagnosis and treatment.

### 2. BACKGROUND OF THE INVENTION

The glycoprotein hormones are a group of evolutionarily conserved hormones involved in the regulation of reproduction and metabolism (Pierce and Parsons, 1981, Endocr. Rev. 11:354-385). This family of hormones includes the follicle-stimulating hormone (FSH), luteinizing hormone (LH), thyroid stimulating hormone (TSH), and chorionic gonadotrophin (CG).

TSH is a 28-30 kDa heterodimeric glycoprotein produced in the thyrotrophs of the anterior pituitary gland. Differences in the molecular weight of TSH are primarily due to the heterogeneity of carbohydrate chains. Its synthesis and secretion is stimulated by thyrotropin-releasing hormone, and inhibited by thyroid hormone in a classical endocrine negative feedback loop. TSH controls thyroid function by interacting with the G protein-coupled TSH receptor (TSHR), (Vassant and Dumont, 1992, Endocr. Rev. 13:596-611). TSH binding to its receptor on thyroid cells leads to the stimulation of second messenger pathways involving predominantly cyclic adenosine 3'5'-monophosphate (cAMP), inositol 1,4,5-triphosphate (IP3) and diacylglycerol (DAG), and ultimately results in the modulation of thyroidal gene expression. Physiological roles of TSH include stimulation of differentiated thyroid functions, such as iodine uptake and organification, the release of thyroid hormone from the gland, and promotion of thyroid growth (Wondisford et al., 1996, Thyrotropin. In: Braverman et al. (eds.), Werner and Ingbar's The Thyroid, Lippencott-Raven, Philadelphia, pp. 190-207).

Structurally, the glycoprotein hormones are related heterodimers comprised of a common α-subunit and a hormone-specific β-subunit. The common human α-subunit contains an apoprotein core of 92 amino acids including 10 half-cystine residues, all of which are in disulfide linkage. It is encoded by a single gene, located on chromosome 6 in humans, and thus identical in amino acid sequence within a given species (Fiddes and Goodman, 1981, J. Mol. Appl. Gen. 1:3-18). The hormone specific β-subunit genes differ in length, structural organization and chromosomal localization (Shupnik et al., 1989, Endocr. Rev. 10:459-475). The human TSH β-subunit gene predicts a mature protein of 118 amino acid residues and is localized on chromosome 1 (Wondisford et al., supra). The various β-subunits can be aligned according to 12 invariant half-cystine residues forming 6 disulfide bonds. Despite a 30 to 80% amino acid sequence identity among the hormones, the β-subunit is sufficiently distinct to direct differential receptor binding with high specificity (Pierce and Parsons, supra).

An important structural component of these hormones is their carbohydrate moiety, which constitutes 15-35% by weight. The common α-subunit has two asparagine (N)-linked oligosaccharides, and the β-subunit one (in TSH and LH) or two (in CG and FSH). In addition, the CG β-subunit has a unique 32 residue carboxyl-terminal extension peptide (CTEP) with four serine (O)-linked glycosylation sites. ( Baenziger, 1994, Glycosylation and glycoprotein hormone function, in Lustbander et aL (eds.) Glycoprotein Hormones: Structure, Function and Clinical Implications. Springer-Verlag, New York, pages 167-174).

Traditionally, structure-function relationships of human glycoprotein hormones have been predominantly performed with gonadotropins, particularly hCG. Recently, the crystal structure of partially deglycosylated hCG has been resolved which revealed two relevant structural features that may also be relevant for the other glycoprotein hormones, (Lapthorn et al., 1994, Nature 369:455-461; Wu et al., 1994, Structure 2:548-558). Both α-subunit and hCG β-subunit have a similar overall topology - each subunit has two β-hairpin loops (LI and L3) on one side of a central cystine knot (formed by three disulfide bonds), and a long loop (L2) on the other.

Molecular biological studies on human TSH have been facilitated by the cloning of TSH β-subunit cDNA and gene (Joshi et al., 1995, Endocrinol. 136:3839-3848), the cloning of TSH receptor cDNA (Parmentier et al., 1989, Science 246:1620-1622; Nagayama et al., 1990, Biochem. Biophys. Res. Commun. 166:394-403), and the recombinant expression of TSH (Cole et al., 1993, Bio/Technol. 11:1014-1024; Grossmann et al., 1995, Mol. Endocrinol. 9:948-958; Szkudlinski et al., 1996 supra). Previous structure-function studies in TSH focussed primarily on the highly conserved regions and the creation of chimeric subunits. However, these approaches did not result in hormones with increased in vitro bioactivity (Grossmann et al., 1997, Endocr. Rev. 18:476-501).

Strategies have been developed to prolong hormonal half life of glycoprotein hormones in circulation. In gene fusion experiments, the carboxyl-terminal extension peptide of the hCG β-subunit, which contains several O-linked carbohydrates, was added to the human TSH β subunit (Joshi et al., 1995, Endocrinol., 136:3839-3848; Grossmann et al., 1997, J. Biol. Chem. 272:21312-21316). Whereas the in vitro activity of these chimeras was not altered, their circulatory half-lives were prolonged, resulting in enhanced in vivo bioactivity. Additionally, expressing the β and α subunits genetically fused as a single chain enhanced stability and a prolonged plasma half-life compared to wild type glycoprotein hormone (Sugahara et al., 1995, Proc. Natl. Acad. Sci. USA 92:2041-2045; Grossmann at al., 1997, J. Biol. Chem. 272:21312-21316).

### 2.1 USE OF TSH IN DIAGNOSIS AND

### MONITORING OF THYROID CARCINOMA

Recombinant TSH has been tested for stimulating ¹³¹I uptake and Tg secretion in the diagnosis and follow up of 19 patients with differentiated thyroid carcinoma, thus avoiding the side effects of thyroid hormone withdrawal (Meier et al., J. Clin. Endocrinol. Metab. 78:188-196). Preliminary results form the first trial are highly encouraging. The incidence of thyroid carcinoma in the United States is approximately 14,000 cases per year. Most of these are differentiated, and papillary or-follicular cancers are the most common subtypes. As the 10- and 20-year survival rate of such differentiated thyroid carcinomas is 90% and 60% respectively, long term monitoring to detect local recurrence and distant metastases becomes essential in the management of such patients, especially since tumor can recur even decades after primary therapy. The principal methods used for follow-up are whole body radioiodine scanning and serum thyroglobulin (Tg) measurements. For optimal sensitivity of these diagnostic procedures, stimulation of residual thyroid tissue by TSH to increase ¹³¹Iodine uptake or Tg secretion, respectively is required. However, post-thyroidectomy thyroid cancer patients are treated with thyroid hormone to suppress endogenous TSH to avoid potential stimulatory effects of TSH on residual thyroid tissue, as well as to maintain euthyroidism. Usually therefore, levo-T₄ or, less commonly used T₃ is withdrawn 4-6 and 2 weeks before radioiodine scanning and Tg determination in order to stimulate endogenous TSH secretion. The accompanying transient but severe hypothyroidism leads to considerable impairment of the quality of life of such patients, and may interfere with their ability to work. Further, since TSH can act as a growth factor for malignant thyroid tissue, prolonged periods of increased endogenous TSH secretion may pose a potential risk for such patients.

In the 1960s, bovine TSH (bTSH) was used to stimulate residual thyroid tissue to overcome the need for elevating endogenous TSH (Blahd et aL, 1960, Cancer 13:745-756). However, several disadvantages soon became apparent leading to the discontinuation of its use in clinical practice. Compared to hormone withdrawal, bTSH proved to be less efficacious in detecting residual malignant thyroid tissue and metastases. In addition, allergic reactions as well as the development of neutralizing antibodies which can further limit the effect of subsequent bTSH administration as well as interference with endogenous TSH determinations were frequently recognized (Braverman et al., 1992, J. Clin. Endocrinol. Metab. 74:1135-1139).

From a diagnostic or therapeutic perspective, there is thus considerable interest for the development of novel hTSH analogs with desirable properties. Hormone activity, in general, can be augmented by the prolongation of hormonal half-life (long acting analogs) or by increasing its intrinsic activity (superactive analogs). The present inventors have made mutant TSH and analogs, and showed that these novel molecules have in vitro and in vivo bioactivity which surpass that of the wild type TSH. Their discovery is described hereinbelow.

### 3. SUMMARY OF THE INVENTION

The present invention relates to mutants of thyroid stimulating hormone (TSH), including mutants of the α subunit common to glycoprotein hormones, mutants of the TSH β subunit, TSH analogs, derivatives, and fragments thereof, preferably TSH heterodimers having mutant subunits with one or more substitutions of amino acid residues and/or TSH heterodimers modified (as described hereinbelow) to increase in vivo half-life. The mutant subunits, mutant TSH heterodimers, TSH analogs, derivatives, and fragments thereof are more active than the wild type TSH in TSH receptor (TSHR) binding and in stimulating TSHR signal transduction, and have prolonged hormonal half lives in circulation.

In a preferred embodiment, the present invention provides a mutant TSH or TSH analog with mutations at amino acid positions located near or within the β hairpin L1 loop of the α subunit and at amino acid positions located near or within the β hairpin L3 loop of the β subunit, and most preferably, the mutant TSH is modified to increase in vivo half life, for example but not limited to, fusing the TSH β subunit to the carboxyl terminal extension peptide (CTEP) of human chorionic gonadotropin (hCG) β subunit, and/or fusing the mutant α and β subunits to produce a single chain TSH analog. The methods of making the foregoing by chemical synthesis or recombinant DNA technology are within the scope of the invention.

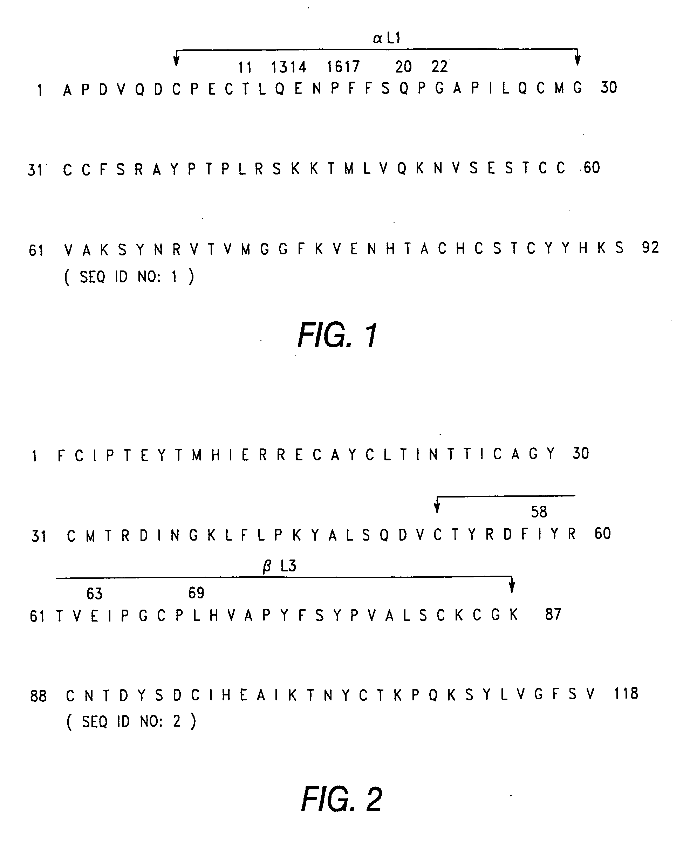

In another preferred embodiment, the present invention provides a mutant α subunit of TSH heterodimer having a mutant α subunit having an amino acid substitution at position 22 of the human α subunit as depicted in Figure 1 (SEQ ID NO:1).

Also provided are nucleic acid sequences encoding mutants of the α and β subunit, and fusion analogs of TSH and its subunits, bioassays, and host-vector systems for hormone production.

The present invention further provides methods of diagnostic and therapeutic uses of mutant TSH and TSH analogs, TSH derivatives, and fragments thereof, in hypothyroidism and cancer, especially thyroid carcinoma. In a preferred embodiment, mutant TSH is used to stimulate radioactive iodine uptake and thyroglobulin secretion in the diagnosis and follow-up monitoring of patients with thyroid cancer. In another preferred embodiment, the mutant TSH heterodimers of the invention can be used in TSH receptor binding inhibition assays to detect the presence of antibodies against the TSH receptor, *e*.*g*., for TSHR autoantibodies characteristic of diseases and disorders, such as but not limited to Graves' Disease. Pharmaceutical and diagnostic compositions comprising mutant TSH or TSH analogs are also provided.

### 3.1 DEFINITIONS

As used herein, the following terms shall have the indicated meanings:
TSH = human thyroid stimulating hormone, unless the species is stated otherwise
TSHR = human thyroid stimulating hormone receptor, unless the species is stated otherwise
hCG = human chorionic gonadotropin
CTEP = carboxyl terminal extension peptide of hCG β subunit
α subunit = human glycoprotein hormone common α subunit, unless stated otherwise
β subunit = human TSHβ subunit, unless stated otherwise

Conventional single letter codes are used to denote amino acid residues.

As used herein, mutations within the TSH subunits are indicated by the TSH subunit, followed by the wild type amino acid residue, the amino acid position, and the mutant amino acid residue. For example, βI58R shall mean a mutation from isoleucine to arginine at position 58 in the human TSH β subunit.

### 4. DESCRIPTION OF THE FIGURES

Figure 1. Amino acid sequence (SEQ ID N0:1) of the human glycoprotein hormone common α subunit. The amino acid residues (positions 8-30) located in or near the αL1 loop is indicated by a line above the sequence. The numbers above the sequence indicate the amino acid positions at which mutation is preferred.
Figure 2. Amino acid sequence (SEQ ID NO:2) of the human TSH β subunit. The amino acid residues (positions 52-87) located in or near the βL3 loop are indicated by a line above the sequence. The numbers above the sequence indicate the amino acid positions at which mutation is preferred.

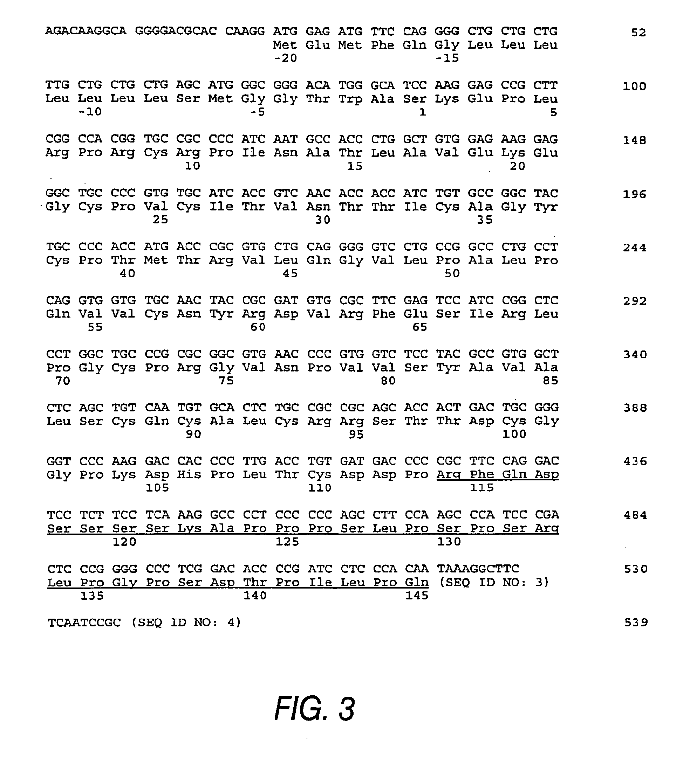

Figure 3. Amino acid sequence of the hCG β subunit. The amino acid residues of the CTEP are indicated by an underline.

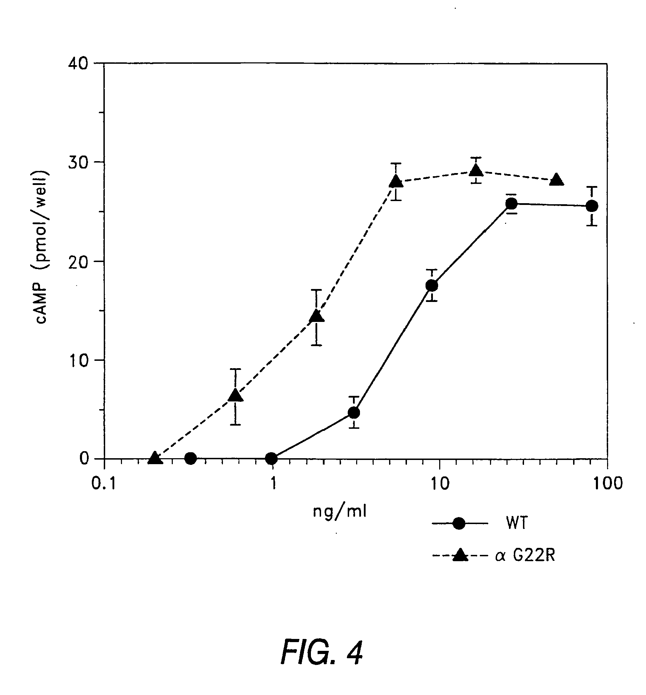

Figure 4. Stimulation of cAMP production in CHO-JP26 cells by a mutant TSH heterodimer (solid triangles) comprising a mutant α subunit with the mutation αG22R and a wild type β subunit; and wild type TSH (solid circles).

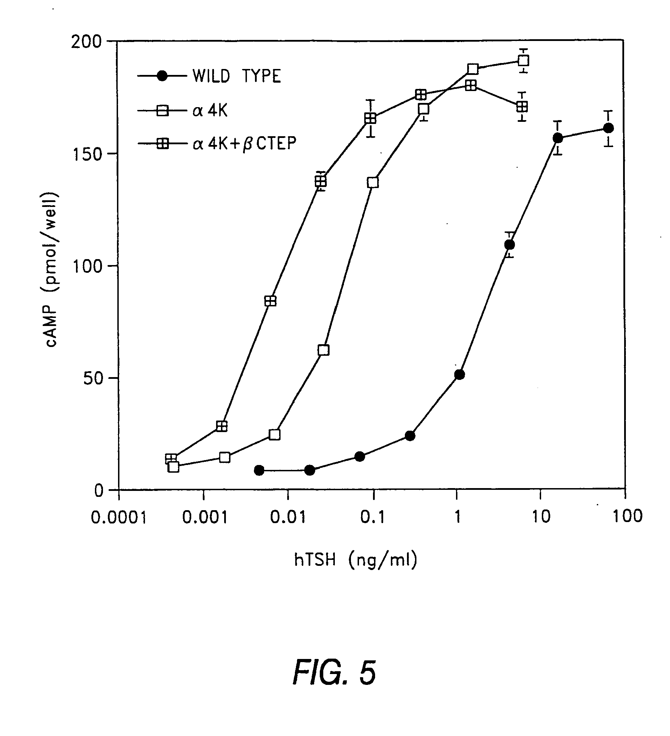

Figure 5. Stimulation of cAMP production in JP09 cells by the mutant TSH heterodimer comprising an α4K subunit with the αQ13K+αE14K+αP16K+αQ20K mutations and a fusion protein of the β subunit and the CTEP (α4K+βCTEP; crossed squares); the mutant TSH heterodimer comprising the αQ13K+αE14K+αP16K+αQ20K mutations and a wild type TSH β subunit (α4K, open squares); and wild type TSH (solid circles).

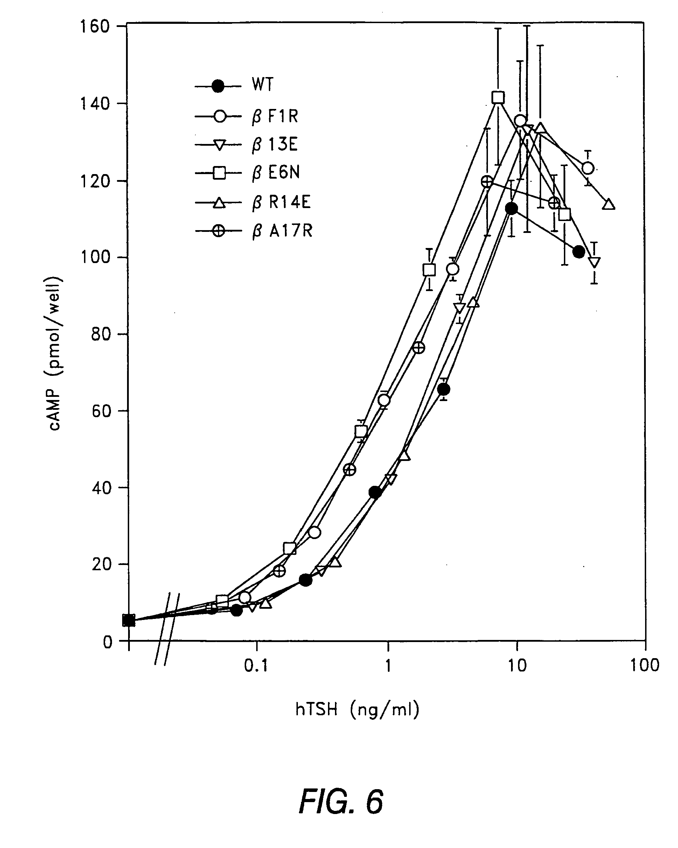

Figure 6. Mutations in the β L1 loop of the TSH-specific β subunit. The line graph illustrates that β13E and βR14E did not have biological activities substantially different from wild type TSH, but that heterodimers incorporating any of the βF1R, βE6N or βA17R mutant subunits exhibited substantially higher biological activities in the standard in vitro assay for cAMP production.

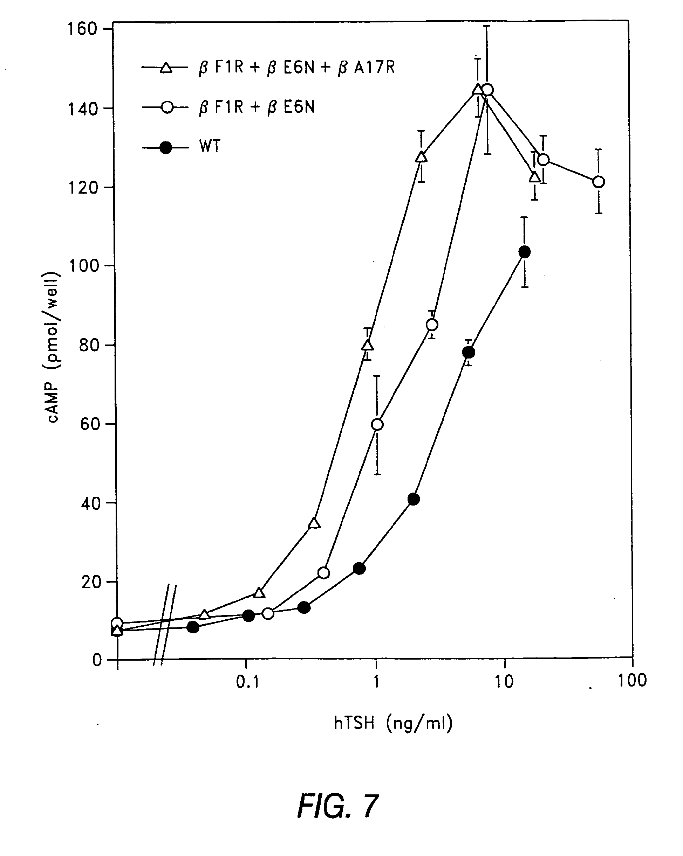

Figure 7. Combinations of mutations in the TSH-specific β subunit exhibited hormone activities substantially greater than wild type TSH in the standard in vitro assay for cAMP production. Mutant heterodimers included either the combination of βF1R and βE6N mutations or the combination of βF1R, βE6N and βA17R mutations.

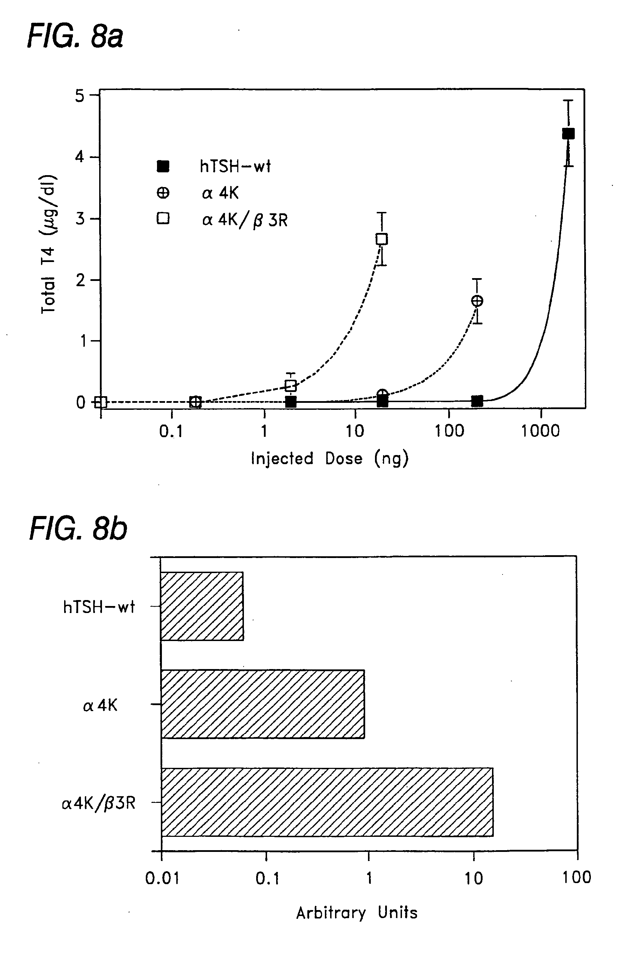

Figure 8A-8B. Line graph showing in vivo activity of hTSH analogs. Figure 8A shows T4 levels in the blood of previously T3-suppressed mice 6 hours after intraperitoneal injection of either hTSH wild type (hTSH-wt) or TSH analogs. Values are the mean ± standard error of the mean of five mice for each data point. Figure 8B is a bar graph showing the total T4 to TSH ratios for individual constructs. Units obtained by dividing serum mean total T4 (µg/dl) by serum mean hTSH (ng/ml) both determined 6 hours after intraperitoneal injection. Recovery of 200 or 20 ng injected material 6 hours after the intraperitoneal injection was similar (2%, 1% and 1% for wild type, α4K, and α4W/β3R, respectively.

### 5. DETAILED DESCRIPTION OF THE INVENTION

The present invention relates to novel mutant TSH proteins, nucleic acid molecules encoding mutant TSH proteins, and methods of making, and diagnostic and therapeutic methods thereof. The present inventors have designed and made mutant thyroid stimulating hormones (TSH), TSH derivatives, TSH analogs, and fragments thereof, that both have mutations (preferably amino acid substitutions) in the α and β subunits that increase the bioactivity of the TSH heterodimer comprised of these subunits relative to the bioactivity of wild type TSH and that are modified to increase the hormonal half life in circulation. The present inventors have found that these mutations to increase bioactivity and the strategies to increase hormonal half life synergize such that TSH heterodimers that have both the superactive mutations and the long acting modifications have much higher bioactivity than would be expected from the sum of the additional activity conferred by the superactive mutations and the long acting modifications individually.

The present inventors have also found that an amino acid substitution at amino acid 22 of the human α subunit (as depicted in Figure 1 (SEQ ID NO:1), preferably a substitution of a basic amino acid, such as lysine or arginine, more preferably arginine, increases the bioactivity of TSH relative to wild type TSH.

The present inventors have designed mutant subunits by combining individual mutations within a single subunit and modifying the subunits and heterodimers to increase the half-life of the heterodimer in vivo (as described herein below). In particular, the inventors have designed mutuant α, mutant β mutant TSH heterodimers having mutations, particularly mutations in specific domains. These domains include the β hairpin L1 loop of the common α subunit (as depicted in Figure 1), and the β hairpin L3 loop of the TSH β subunit (as depicted in Figure 2). In one embodiment, the present invention provides mutant α subunits, mutant TSH β subunits, and TSH heterodimers comprising either one mutant α subunit or one mutant β subunit, wherein the mutant α subunit comprises single or multiple amino acid substitutions, preferably located within or near the β hairpin L1 loop of the α subunit, and wherein the mutant β subunit comprises single or multiple amino acid substitutions, preferably located in or near the β hairpin L3 loop of the β subunit (preferably, these mutations increase bioactivity of the TSH heterodimer comprising the mutant subunit and the TSH heterodimer having the mutant subunit has also been modified to increase the serum half-life relative to the wild-type TSH heterodimer).

According to the invention, a mutant β subunit comprising single or multiple amino acid substitutions, preferably located in or near the β hairpin L3 loop of the β subunit, can be fused at its carboxyl terminal to the CTEP. Such a mutant β subunit-CTEP subunit may be coexpressed and/or assembled with either a wild type or mutant α subunit to form a functional TSH heterodimer which has a bioactivity and a serum half life greater than wild type TSH.

In another embodiment, a mutant β subunit comprising single or multiple amino acid substitutions, preferably located in or near the β hairpin L3 loop of the β subunit, and mutant α subunit comprising single or multiple amino acid substitutions, preferably located in or near the β hairpin L1 loop of the α subunit, are fused to form a single chain TSH analog. Such a mutant β subunit-mutant α subunit fusion has a bioactivity and serum half-life greater than wild type TSH.

In yet another embodiment, mutant β subunit comprising single or multiple amino acid substitutions, preferably located in or near the β hairpin L3 loop of the β subunit, and further comprising the CTEP in the carboxyl terminus, and mutant α subunit comprising single or multiple amino acid substitutions, preferably located in or near the β hairpin L1 loop of the α subunit, are fused to form a single chain TSH analog.

Fusion proteins, analogs, and nucleic acid molecules encoding such proteins and analogs, and production of the foregoing proteins and analogs, e.g., by recombinant DNA methods, are also provided.

In particular aspects, the invention provides amino acid sequences of mutant α and β subunits, and fragments and derivatives thereof which are otherwise functionally active. "Functionally active" mutant TSH α and β subunits as used herein refers to that material displaying one or more known functional activities associated with the wild-type subunit, *e*.*g*., binding to the TSHR, triggering TSHR signal transduction, antigenicity (binding to an anti-TSH antibody), immunogenicity, etc.

In specific embodiments, the invention provides fragments of mutant α and TSH β subunits consisting of at least 6 amino acids, 10 amino acids, 50 amino acids, or of at least 75 amino acids. In various embodiments, the mutant α subunits comprise or consist essentially of a mutated αL1 loop domain; the mutant β subunits comprise or consist essentially of a mutated βL3 loop domain.

The present invention further provides nucleic acid sequences encoding mutant α and mutant β subunits and modified mutant α and β subunits (*e*.*g*. mutant β subunit-CTEP fusions or mutant β subunit-mutant α subunit fusions), and methods of using the nucleic acid sequences. The mutations in the α subunits and β subunits are described in greater detail respectively in Section 5.1 and 5.2 hereinbelow.

The present invention also relates to therapeutic and diagnostic methods and compositions based on mutant α subunits, mutant β subunits, mutant TSH heterodimers, and TSH analogs, derivatives, and fragments thereof. The invention provides for the use of mutant TSH and analogs of the invention in the diagnosis and treatment of thyroid cancer by administering mutant TSH and analogs that are more active and have a longer half life in circulation than the wild type TSH. The invention further provides methods of diagnosing diseases and disorders characterized by the presence of autoantibodies against the TSH receptor using the mutant TSH heterodimers and analogs of the invention in TSH receptor binding inhibition assays. Diagnostic kits are also provided by the invention.

The invention also provides methods of treatment of disorders of the thyroid gland, such as thyroid cancer.

For clarity of disclosure, and not by way of limitation, the detailed description of the invention is divided into the subsections which follow.

### 5.1 MUTANTS OF THE COMMON α SUBUNIT

The common human α subunit of glycoprotein hormones contains 92 amino acids as depicted in Figure 1 (SEQ ID NO: 1), including 10 half-cysteine residues, all of which are in disulfide linkages. The invention relates to mutants of the α subunit of human glycoprotein hormones wherein the subunit comprises single or multiple amino acid substitutions, preferably located in or near the β hairpin L1 loop of the α subunit. The amino acid residues located in or near the αL1 loop, starting from position 8-30 as depicted in Figure 1 are found to be important in effecting receptor binding and signal transduction. Amino acid residues located in the αL1 loop, such as those at position 11-22, form a cluster of basic residues in all vertebrates except hominoids, and have the ability to promote receptor binding and signal transduction. In particular, the amino acid residue at position 22 is found to be one of the residues that influence the potency of TSH.

According to the invention, the mutant α subunits have substitutions, deletions or insertions, of one, two, three, four, or more amino acid residues in the wild type protein.

In one embodiment, the mutant α subunits have one or more substitutions of amino acid residues relative to the wild type α subunit, preferably, one or more amino acid substitutions in the amino acid residues selected from among residues at position 8-30, 11-22, 8-22, 11-30, 11-16, 14-22, 13-14 or 16-17.

In another embodiment of the invention, the mutant α subunit has a single amino acid substitution at positions 11, 13, 14, 16, 17, 20 or 22 of the α subunit sequence. In yet another embodiment of the invention, the mutant α subunit has multiple amino acid substitutions in the amino acid residues selected from among residues at positions 11, 13, 14, 16, 17, 20 and 22 of the α subunit sequence.

In various embodiments, the amino acid substitution is made with a positively charged residue or basic residue from the group consisting of lysine, arginine and, less preferably, histidine.

In a preferred embodiment, the mutant α subunit of the invention has a single amino acid substitution at position 22, wherein a glycine residue is substituted with an arginine, i.e., αG22R. A mutant α subunit having the αG22R mutation may have at least one or more additional amino acid substitutions, such as but not limited to αT11K, αQ13K, αE14K, αP16K, αF17R, and αQ20K. In other preferred embodiments, the mutant α subunit has one, two, three, four, or more of the amino acid substitutions selected from the group consisting of αT11K, αQ13K, αE14K, αP16K, αF17R, αQ20K, and αG22R. For example, one of the preferred mutant α subunit (to be used in conjunction with a modification to increase the serum half-life of the TSH heterodimer having the mutant α subunit), also referred to herein as α4K, comprises four mutations: αQ13K+αE14K+αP16K+αQ20K.

The mutant α subunits of the invention are functionally active, i.e., capable of exhibiting one or more functional activities associated with the wild-type α subunit. Preferably, the mutant α subunit is capable of noncovalently associating with a wild type or mutant β subunit to form a TSH heterodimer that binds to the TSHR. Preferably, such a TSH heterodimer also triggers signal transduction. Most preferably, such a TSH heterodimer comprising a mutant α subunit has an in vitro bioactivity and/or in vivo bioactivity greater than the wild type TSH. It is contemplated in the present invention that more than one mutation can be combined within a mutant α subunit to make a superactive α mutant, which in association with a wild type or mutant β subunit, forms a TSH heterodimer, that has a significant increase in bioactivity relative to the wild type TSH. It is also contemplated that the α subunit mutations will be combined with strategies to increase the serum half-life of the TSH heterodimer having the mutant α subunit (*i*.*e*. a TSH heterodimer having a β subunit-CTEP fusion or a β subunit-α subunit fusion). The mutations within a subunit and the long acting modifications act synergistically to produce an unexpected increase in the bioactivity.

As another example, such mutant α subunits which have the desired immunogenicity or antigenicity can be used, for example, in immunoassays, for immunization, for inhibition of TSHR signal transduction, etc. Mutant α subunit can be tested for the desired activity by procedures known in the art, including but not limited to the assays described in Section 5.8.

### 5.2 MUTANTS OF THE TSH β SUBUNIT

The common human β subunit of glycoprotein hormones contains 118 amino acids as depicted in Figure 2 (SEQ ID No: 2). The invention relates to mutants of the β subunit of TSH wherein the subunit comprises single or multiple amino acid substitutions, preferably located in or near the β hairpin L3 loop of the β subunit, where such mutant β subunits are fused to CTEP of the β subunit of hCG or are part of a TSH heterodimer having a mutant α subunit with an amino acid substitution at position 22 (as depicted in Figure 1 (SEQ ID NO: 1)), or being an α subunit-β subunit fusion. The amino acid residues located in or near the βL3 loop at positions 52-87 of the human TSH β subunits are mapped to amino acid residues in hCG that are located peripherally and appear to be exposed to the surface in the crystal structure. Of particular interest is a cluster of basic residues in hCG which is not present in TSH (starting from position 58-69). Substitution of basic or positively charged residues into this domain of human TSH leads to an additive and substantial increase in TSHR binding affinity as well as intrinsic activity.

The mutant TSH heterodimers of the invention have β subunits having substitutions, deletions or insertions, of one, two, three, four, or more amino acid residues in the wild type subunit.

In one embodiment, the mutant β subunit has one or more substitutions of amino acid residues relative to the wild type β subunit, preferably, one or more amino acid substitutions in the amino acid residues selected from among residues at position 52-87, 52-69, 58-69 or 58-87 of the β subunit as depicted in Figure 2 (SEQ ID NO: 2).

In another embodiment of the invention, the mutant β subunit has a single amino acid substitution at positions 58, 63 or 69 of the β subunit sequence as depicted in Figure 2 (SEQ ID NO: 2). In yet another embodiment of the invention, the mutant β subunit has multiple amino acid substitutions in the amino acid residues selected from among residues at positions 58, 63 or 69 of the β subunit sequence as depicted in Figure 2 (SEQ ID NO: 2).

In various embodiments, the amino acid substitution is made with a positively charged residue or basic residue from the group consisting of lysine, arginine, and, less preferably, histidine.

In a preferred embodiment, the mutant β subunit has one, two, three, or more of the amino acid substitutions selected from the group consisting of βI58R, βE63R, and βL69R. For example, one of the preferred mutant β subunit, also referred to herein as β3R, comprises three mutations: βI58R+βE63R+βL69R.

The mutant TSH, TSH analogs, derivatives, and fragments thereof of the invention having mutant β subunits either also have a mutant α subunit with an amino acid substitution at position 22 (as depicted in Figure 1 (SEQ ID NO: 1)) and/or have a serum half life that is greater than wild type TSH. In one embodiment, a mutant β subunit comprising one or more substitutions of amino acid residues relative to the wild type β subunits is covalently bound to the carboxyl terminal extension peptide (CTEP) of hCG. The CTEP, which comprises the carboxyl terminus 32 amino acids of the hCG β subunit (as depicted in Figure 3), is covalently bound to the mutant β subunit, preferably the carboxyl terminus of the mutant β subunit is covalently bound to the amino terminus of CTEP. The β subunit and the CTEP may be covalently bound by any method known in the art, *e*.*g*., by a peptide bond or by a heterobifunctional reagent able to form a covalent bond between the amino terminus and carboxyl terminus of a protein, for example but not limited to, a peptide linker. In a preferred embodiment, the mutant β subunit and CTEP are linked via a peptide bond. In various preferred embodiments, the mutant β subunit-CTEP fusions may comprise one, two, three, or more of the amino acid substitutions selected from the group consisting of βI58R, βE63R, and βL69R.

In another embodiment, a mutant β subunit is fused, *i*.*e*. covalently bound, to an α subunit, preferably a mutant α subunit (*e*.*g*. as described in Section 5.2 *supra*).

The mutant β subunits of the invention are functionally active, i.e., capable of exhibiting one or more functional activities associated with the wild-type β subunit. Preferably, the mutant β subunit is capable of noncovalently associating with a wild type or mutant α subunit to form a TSH heterodimer that binds to the TSHR. Preferably, such a TSH heterodimer also triggers signal transduction. Most preferably, such a TSH heterodimer comprising a mutant β subunit has an in vitro bioactivity and/or in vivo bioactivity greater than the bioactivity of wild type TSH. It is contemplated in the present invention that more than one mutation can be combined within a mutant β subunit to make a mutant TSH heterodimer that has a significant increase in bioactivity relative to the wild type TSH. The inventors discovered that multiple mutations within a subunit and modifications to increase the half-life of the TSH heterodimer (*i*.*e*. the β subunit-CTEP fusion and/or the β subunit-α subunit fusion) can act synergistically to achieve bioactivity that is greater than the sum of the increase of the mutations and the long acting modifications.

Mutant β subunit can be tested for the desired activity by procedures known in the art, including but not limited to the assays described in Section 5.8.

### 5.3 MUTANT TSH HETERODIMERS AND TSH ANALOGS

The present invention provides mutant human TSH heterodimers and human TSH analogs comprising a mutant α subunit and a mutant β subunit, wherein the mutant α subunit comprises single or multiple amino acid substitutions, preferably located in or near the β hairpin L1 loop of the α subunit (as described in Section 5.1), and the mutant β subunit comprises single or multiple amino acid substitutions, preferably located in or near the β hairpin L3 loop of the β subunit (as described in Section 5.2), which heterodimer or analog also is modified to increase the serum half-life (*e*.*g*. by β subunit-CTEP fusion or by α subunit-β subunit fusion). The single or multiple amino acid substitutions in the mutant α subunit can be made in amino acid residues selected from among positions 8-30, and preferably positions 11-22, of the amino acid sequence of human α subunit. The single or multiple amino acid substitutions in the mutant TSH β subunit can be made in amino acid residues selected from among positions 52-87, and preferably positions 58-69, of the amino acid sequence of human TSH β subunit. A non-limiting example of such a mutant TSH comprises a heterodimer of the mutant α subunit, α4K, and the mutant β subunit, β3R, as described above.

In one embodiment, the invention provides TSH heterodimers comprising an α subunit, preferably a mutant α subunit, and a β subunit, preferably a mutant β subunit, wherein either the mutant α or mutant β subunit is fused to the CTEP of the β subunit of hCG (as described in Section 5.2). The term fusion protein refers herein to a protein which is the product of the covalent bonding of two peptides. Covalent bonding includes any method known in the art to bond two peptides covalently at their amino- and carboxyl- termini, respectively, such methods include but are not limited to, joining via a peptide bond or via a heterobifunctional reagent, for example but not by way of limitation, a peptide linker. In a preferred embodiment, the mutant TSH heterodimer may comprise a mutant human α subunit and a mutant human TSH β subunit, wherein the mutant human TSH β subunit is covalently bound at its carboxyl terminus to the amino terminus of CTEP.

The present invention also relates to single chain human TSH analogs comprising a mutant human α subunit covalently bound (as described above for the β subunit-CTEP fusion) to a mutant human TSH β subunit wherein the mutant α subunit and the mutant human TSH β subunit each comprise at least one amino acid substitution in the amino acid sequence of the respective subunit. In a preferred embodiment, the mutant β subunit is joined via a peptide linker to a mutant α subunit. In a more preferred embodiment, the CTEP of hCG, which has a high serine/proline content and lacks significant secondary structure, is the peptide linker.

Preferably, the mutant α subunit comprising single or multiple amino acid substitutions, preferably located in or near the β hairpin L1 loop of the α subunit (as described in Section 5.1, *supra)* is covalently bound to a mutant β subunit comprising single or multiple amino acid substitutions, preferably located in or near the β hairpin L3 loop of the β subunit (as described in Section 5.2, *supra*).

In one embodiment, the mutant human TSH β subunit comprising at least one amino acid substitution in amino acid residues selected from among positions 52-87, preferably positions 58-69, of the amino acid sequence of human TSH β subunit is covalently bound at its carboxyl terminus with the amino terminus of a wild type human TSH α subunit or a mutant TSH α subunit comprising at least one amino acid substitution, wherein the one or more substitutions are in amino acid residues selected from among positions 8-30, and preferably 11-22, of the amino acid sequence of human α subunit.

The mutant α subunit or mutant human TSH β subunit may each lack its signal sequence.

The present invention also provides a human TSH analog comprising a mutant human TSH β subunit covalently bound to CTEP which is, in turn, covalently bound to a mutant α subunit, wherein the mutant α subunit and the mutant human TSH β subunit each comprise at least one amino acid substitution in the amino acid sequence of each of the subunits.

In a specific embodiment, a mutant β subunit-CTEP fusion is covalently bound to a mutant α subunit, such that the carboxyl terminus of the mutant β subunit is linked to the amino terminal of the mutant α subunit through the CTEP of hCG. Preferably, the carboxyl terminus of a mutant β subunit is covalently bound to the amino terminus of CTEP, and the carboxyl terminus of the CTEP is covalently bound to the amino terminal of a mutant α subunit without the signal peptide.

Accordingly, in a specific embodiment, the human TSH analog comprises a mutant human TSH β subunit comprising at least one amino acid substitution in amino acid residues selected from among positions 58-69 of the amino acid sequence of human TSH β subunit covalently bound at the carboxyl terminus of the mutant human TSH β subunit with the amino terminus of CTEP which is joined covalently at the carboxyl terminus of said carboxyl terminal extension peptide with the amino terminus of a mutant α subunit comprising at least one amino acid substitution, wherein the one or more substitutions are in amino acid residues selected from among positions 11-22 of the amino acid sequence of human α subunit.

In another preferred embodiment, the mutant TSH heterodimer comprises a mutant α subunit having an amino acid substitution at position 22 of the human α subunit sequence (as depicted in Figure 1 (SEQ ID NO:1)), preferably a substitution with a basic amino acid (such as arginine, lysine, and less preferably, histidine), more preferably with arginine.

In specific embodiments, the mutant TSH heterodimer comprising at least one mutant subunit or the single chain TSH analog as described above is functionally active, i.e., capable of exhibiting one or more functional activities associated with the wild-type TSH, such as TSHR binding, TSHR signalling and extracellular secretion. Preferably, the mutant TSH heterodimer or single chain TSH analog is capable of binding to the TSHR, preferably with affinity greater than the wild type TSH. Also it is preferable that such a mutant TSH heterodimer or single chain TSH analog triggers signal transduction. Most preferably, the mutant TSH heterodimer comprising at least one mutant subunit or the single chain TSH analog of the present invention has an in vitro bioactivity and/or in vivo bioactivity greater than the wild type TSH and has a longer serum half-life than wild type TSH. Mutant TSH heterodimers and single chain TSH analogs of the invention can be tested for the desired activity by procedures known in the art, including but not limited to the assays described in Section 5.8. Working examples of mutant TSH heterodimers are described in Section 6.

### 5.4 POLYNUCLEOTIDES ENCODING MUTANT TSH AND ANALOGS

The present invention also relates to nucleic acids molecules comprising sequences encoding mutant subunits of human TSH and TSH analogs of the invention, wherein the sequences contain at least one base insertion, deletion or substitution, or combinations thereof that results in single or multiple amino acid additions, deletions and substitutions relative to the wild type TSH. Base mutation that does not alter the reading frame of the coding region is preferred. As used herein, when two coding regions are said to be fused, the 3' end of one nucleic acid molecule is ligated to the 5' (or through a nucleic acid encoding a peptide linker) end of the other nucleic acid molecule such that translation proceeds from the coding region of one nucleic acid molecule into the other without a frameshift.

Due to the degeneracy of nucleotide coding sequences, any other DNA sequences that encode the same amino acid sequence for a mutant α or β subunit may be used in the practice of the present invention. These include but are not limited to nucleotide sequences comprising all or portions of the coding region of the α or β subunit which are altered by the substitution of different codons that encode the same amino acid residue within the sequence, thus producing a silent change.

In one embodiment, the present invention provides nucleic acid molecules comprising sequences encoding mutant α subunits, wherein the mutant α subunits comprise single or multiple amino acid substitutions, preferably located in or near the β hairpin L1 loop of the α subunit (as described in Section 5.1). In a specific embodiment, the invention provides nucleic acids encoding mutant α subunits having an amino acid substitution at position 22 of the amino acid sequence of the α subunit as depicted in Figure 1 (SEG ID NO:1), preferably substitution with a basic amino acid, more preferably substitution with arginine. The present invention further provides nucleic acids molecules comprising sequences encoding mutant β subunits comprising single or multiple amino acid substitutions, preferably located in or near the β hairpin L3 loop of the β subunit, and/or covalently joined to CTEP (as described in Section 5.2).

In yet another embodiment, the invention provides nucleic acid molecules comprising sequences encoding single chain TSH analogs, such as those described in Section 5.3, wherein the coding region of a mutant α subunit comprising single or multiple amino acid substitutions, preferably located in or near the β hairpin L1 loop of the α subunit, is fused with the coding region of a mutant β subunit comprising single or multiple amino acid substitutions, preferably located in or near the β hairpin L3 loop of the β subunit. Also provided are nucleic acid molecules encoding a single chain TSH analog wherein the carboxyl terminus of the mutant β subunit is linked to the amino terminus of the mutant α subunit through the CTEP of the β subunit of hCG. In a preferred embodiment, the nucleic acid molecule encodes a single chain TSH analog, wherein the carboxyl terminus of a mutant β subunit is covalently bound to the amino terminus of CTEP, and the carboxyl terminus of the CTEP is covalently bound to the amino terminus of a mutant α subunit without the signal peptide.

The single chain analogs of the invention can be made by ligating the nucleic acid sequences encoding the mutant α and β subunits to each other by methods known in the art, in the proper coding frame, and expressing the fusion protein by methods commonly known in the art. Alternatively, such a fusion protein may be made by protein synthetic techniques, *e*.*g*., by use of a peptide synthesizer.

### 5.5 PREPARATION OF MUTANT TSH SUBUNITS AND ANALOGS

The production and use of the mutant α subunits, mutant β subunits, mutant TSH heterodimers, TSH analogs, single chain analogs, derivatives and fragments thereof of the invention are within the scope of the present invention. Described herein are methods for making the foregoing.

### 5.5.1 TSH GENE CLONING

The nucleotide sequences of the cDNA and the gene encoding the human common α subunit (Fiddes and Goodman, 1979, Nature 281:351-356; Fiddes and Goodman, 1981, J. Mol. Appl. Gen. 1:3-18), and the human TSH β subunit (Hayashizaki et al., 1985, FEBS Lett, 88:394-400; Wondisford et al., 1988, J. Bio. Chem. 263:12538-12542; Wondisford et al., 1988, Mol. Endocrinol. 2:32-39) are published.

Coding regions for the subunits can be obtained by standard procedures known in the art from cloned DNA (e.g., a DNA "library"), by chemical synthesis, by cDNA cloning, or by the cloning of genomic DNA, or fragments thereof, purified from the desired cell (see, for example, Sambrook et al., 1989, Molecular Cloning, A Laboratory Manual, 2d Ed., Cold Spring Harbor Laboratory Press, Cold Spring Harbor, New York; Glover, D.M. (ed.), 1985, DNA Cloning: A Practical Approach, MRL Press, Ltd., Oxford, U.K. Vol. I, II.) Polymerase chain reaction (PCR) can be used to amplify sequences encoding the common α or TSH β subunits in a genomic or cDNA library. Synthetic oligonucleotides may be utilized as primers to amplify by PCR sequences from a source (RNA or DNA), preferably a cDNA library. The DNA being amplified can include cDNA or genomic DNA from any human. After successful isolation or amplification of a segment of a subunit, that segment may be molecularly cloned and sequenced, and utilized as a probe to isolate a complete cDNA or genomic clone. This, in turn, will permit the characterization the gene's nucleotide sequence, and the production of its protein product for functional analysis and/or therapeutic or diagnostic use, as described *infra.*

Alternatives to isolating the coding regions for the subunits include, but are not limited to, chemically synthesizing the gene sequence itself from the published sequence. Other methods are possible and within the scope of the invention. The above-methods are not meant to limit the following general description of methods by which mutants of the hormone subunits may be obtained.

The identified and isolated gene can be inserted into an appropriate cloning vector for amplification of the gene sequence. A large number of vector-host systems known in the art may be used. Possible vectors include, but are not limited to, plasmids or modified viruses, but the vector system must be compatible with the host cell used. Such vectors include, but are not limited to, bacteriophages such as lambda derivatives, or plasmids such as pBR322 or pUC plasmid derivatives or the BLUESCRIPT vector (Stratagene). The insertion into a cloning vector can, for example, be accomplished by ligating the DNA fragment into a cloning vector which has complementary cohesive termini. However, if the complementary restriction sites used to fragment the DNA are not present in the cloning vector, the ends of the DNA molecules may be enzymatically modified. Alternatively, any site desired may be produced by ligating nucleotide sequences (linkers) onto the DNA termini; these ligated linkers may comprise specific chemically synthesized oligonucleotides encoding restriction endonuclease recognition sequences. In an alternative method, the cleaved vector and mutant subunit gene may be modified by homopolymeric tailing. Recombinant molecules can be introduced into host cells via transformation, transfection, infection, electroporation, etc., so that many copies of the gene sequence are generated.

In an alternative method, the desired gene may be identified and isolated after insertion into a suitable cloning vector in a "shot gun" approach. Enrichment for the desired gene, for example, by size fractionation, can be done before insertion into the cloning vector.

In specific embodiments, transformation of host cells with recombinant DNA molecules that comprise the mutant subunit gene, cDNA, or synthesized DNA sequence enables generation of multiple copies of the gene. Thus, the gene may be obtained in large quantities by growing transformants, isolating the recombinant DNA molecules from the transformants and, when necessary, retrieving the inserted gene from the isolated recombinant DNA. Copies of the gene are used in mutagenesis experiments to study the structure and function of mutant subunits, TSH heterodimers and TSH analogs.

### 5.5.2 MUTAGENESIS

The mutations present in mutant α or β subunits, mutant TSH heterodimers, TSH analogs, single chain TSH analogs of the invention can be produced by various methods known in the art. The manipulations which result in their production can occur at the gene or protein level. For example, the cloned coding region of the subunits can be modified by any of numerous strategies known in the art (Sambrook et al., 1990, Molecular Cloning, A Laboratory Manual, 2d ed., Cold Spring Harbor Laboratory, Cold Spring Harbor, New York). The sequence can be cleaved at appropriate sites with restriction endonuclease(s), followed by further enzymatic modification if desired, isolated, and ligated *in vitro.* In the production of a mutant subunit, care should be taken to ensure that the modified gene remains within the same translational reading frame, uninterrupted by translational stop signals, in the gene region where the subunit is encoded.

Additionally, the nucleic acid sequence encoding the subunits can be mutated *in vitro* or *in vivo,* to create variations in coding regions (*e*.*g*. amino acid substitutions), and/or to create and/or destroy translation, initiation, and/or termination sequences, and/or form new restriction endonuclease sites or destroy preexisting ones, to facilitate further *in vitro* modification. Any technique for mutagenesis known in the art can be used, including but not limited to, chemical mutagenesis, *in vitro* site-directed mutagenesis (Hutchinson, C., et al., 1978, J. Biol. Chem 253:6551), PCR-based overlap extension (Ho et al., 1989, Gene 77:51-59), PCR-based megaprimer mutagenesis (Sarkar et al., 1990, Biotechniques, 8:404-407), etc. Mutations can be confirmed by double stranded dideoxy DNA sequencing.

One or more amino acid residues within a subunit can be substituted by another amino acid, preferably with different properties, in order to generate a range of functional differentials. Substitutes for an amino acid within the sequence may be selected from members of a different class to which the amino acid belongs. The nonpolar (hydrophobic) amino acids include alanine, leucine, isoleucine, valine, proline, phenylalanine, tryptophan and methionine. The polar neutral amino acids include glycine, serine, threonine, cysteine, tyrosine, asparagine, and glutamine. The positively charged (basic) amino acids include arginine, lysine and histidine. The negatively charged (acidic) amino acids include aspartic acid and glutamic acid.

Manipulations of the mutant subunit sequence may also be made at the protein level. Included within the scope of the invention are mutant subunits, mutant TSH heterodimer, TSH analogs, single chain analogs which are differentially modified during or after translation, *e.g.,* by glycosylation, acetylation, phosphorylation, amidation, derivatization by known protecting/blocking groups, proteolytic cleavage, linkage to an antibody molecule or other cellular ligand, etc. Any of numerous chemical modifications may be carried out by known techniques, including but not limited to specific chemical cleavage by cyanogen bromide, trypsin, chymotrypsin, papain, V8 protease, NaBH₄; acetylation, formylation, oxidation, reduction; metabolic synthesis in the presence of tunicamycin; etc.

In addition, mutant subunits and single chain TSH analogs can be chemically synthesized. For example, a peptide corresponding to a portion of a mutant subunit which comprises the desired mutated domain can be synthesized by use of a peptide synthesizer. Furthermore, if desired, nonclassical amino acids or chemical amino acid analogs can be introduced as a substitution or addition into the mutant subunit sequence. Non-classical amino acids include but are not limited to the D-isomers of the common amino acids, α-amino isobutyric acid, 4-aminobutyric acid, Abu, 2-amino butyric acid, γ-Abu, ∈-Ahx, 6-amino hexanoic acid, Aib, 2-amino isobutyric acid, 3-amino propionic acid, ornithine, norleucine, norvaline, hydroxyproline, sarcosine, citrulline, cysteic acid, t-butylglycine, t-butylalanine, phenylglycine, cyclohexylalanine, β-alanine, fluoro-amino acids, designer amino acids such as β-methyl amino acids, Cα-methyl amino acids, Nα-methyl amino acids, and amino acid analogs in general. Furthermore, the amino acid can be D (dextrorotary) or L (levorofary).

In specific embodiments, the mutant subunit or TSH analog is a fusion protein either comprising, for example, but not limited to, a mutant β subunit and the CTEP of the β subunit of hCG or a mutant β subunit and a mutant α subunit. In one embodiment, such a fusion protein is produced by recombinant expression of a nucleic acid encoding a mutant or wild type subunit joined in-frame to the coding sequence for another protein, such as but not limited to toxins, such as ricin or diphtheria toxin. Such a fusion protein can be made by ligating the appropriate nucleic acid sequences encoding the desired amino acid sequences to each other by methods known in the art, in the proper coding frame, and expressing the fusion protein by methods commonly known in the art. Alternatively, such a fusion protein may be made by protein synthetic techniques, *e.g.,* by use of a peptide synthesizer. Chimeric genes comprising portions of mutant α and/or β subunit fused to any heterologous protein-encoding sequences may be constructed. A specific embodiment relates to a single chain analog comprising a mutant α subunit fused to a mutant β subunit, preferably with a peptide linker between the mutant α subunit and the mutant β subunit.

### 5.6 EXPRESSION OF THE MUTANT SUBUNIT GENES

The nucleotide sequence coding for a mutant subunit of TSH, or a functionally active analog or fragment or other derivative thereof (see Section 5.4), can be inserted into an appropriate expression vector, *i.e.,* a vector which contains the necessary elements for the transcription and translation of the inserted protein-coding sequence. The necessary transcriptional and translational signals can also be supplied by the native common α subunit cDNA or gene, or the human TSH β subunit cDNA or gene, and/or genomic sequences flanking each of the two genes. A variety of host-vector systems may be utilized to express the protein-coding sequence. These include but are not limited to mammalian cell systems infected with virus (e.g., vaccinia virus, adenovirus, etc.); insect cell systems infected with virus (*e*.*g*., baculovirus); microorganisms such as yeast containing yeast vectors. The expression elements of vectors vary in their strengths and specificities. Depending on the host-vector system utilized, any one of a number of suitable transcription and translation elements may be used. In specific embodiments, a mutant human α subunit coding region and/or a human mutant TSH β subunit coding region, or a sequence encoding a mutated and functionally active portion of the respective mutant subunit is expressed.

Any of the methods previously described for the insertion of DNA fragments into a vector may be used to construct expression vectors containing a chimeric gene consisting of appropriate transcriptional/translational control signals and the protein coding sequences. These methods may include *in vitro* recombinant DNA and synthetic techniques and *in vivo* recombinants (genetic recombination). Expression of nucleotide sequence encoding a mutant α subunit and/or a mutant TSH β subunit or peptide fragments thereof may be regulated by a second nucleotide sequence so that the mutant subunit(s) or peptide is expressed in a host transformed with the recombinant DNA molecule. For example, expression of a mutant α subunit and/or a mutant TSH β subunit or peptide fragments thereof may be controlled by any promoter/enhancer element known in the art. Promoters which may be used to include, but are not limited to, the SV40 early promoter region (Bemoist and Chambon, 1981, Nature 290:304-310), the promoter contained in the 3' long terminal repeat of Rous sarcoma virus (Yamamoto, et al., 1980, Cell 22:787-797), the herpes thymidine kinase promoter (Wagner et aL, 1981, Proc. Natl. Acad. Sci. U.S.A. 78:1441-1445), the regulatory sequences of the metallothionein gene (Brinster et al., 1982, Nature 296:39-42).

In a specific embodiment, a vector is used that comprises one or more promoters operably linked to the coding region of a mutant α subunit or a mutant TSH β subunit or both, one or more origins of replication, and, optionally, one or more selectable markers (*e*.*g*., an antibiotic resistance gene). Expression of the two subunits within the same eukaryotic host cell is preferred as such coexpression favors proper assembly and glycosylation of a functional TSH heterodimer. Thus, in a preferred embodiment, such vectors are used to express both the mutant α and the mutant β subunits in a host cell. The coding region of each of the mutant subunits may be cloned into separate vectors; the vectors being introduced into a host cell sequentially or simultaneously. Alternatively, the coding regions of both subunits may be inserted in one vector to which the appropriate promoters are operably linked.

A host cell strain may be chosen which modulates the expression of the inserted sequences, or modifies and processes the gene product in the specific fashion desired. Expression from certain promoters can be elevated in the presence of certain inducers; thus, expression of the genetically engineered mutant subunits may be controlled. Furthermore, different host cells have characteristic and specific mechanisms for the translational and post-translational processing and modification (*e*.*g*., glycosylation, phosphorylation of proteins). Appropriate cell lines or host systems can be chosen to ensure the desired modification and processing of the foreign protein expressed. Expression in mammalian cells can be used to ensure "native" glycosylation of a heterologous protein. Furthermore, different vector/host expression systems may effect processing reactions to different extents.

Once a recombinant host cell which expresses the mutant TSH α and/or β subunit gene sequence is identified, the gene product can be analyzed. This is achieved by assays based on the physical or functional properties of the product, including radioactive labelling of the product followed by analysis by gel electrophoresis, immunoassay, etc.

### 5.7 GENERATION OF ANTIBODIES TO MUTANT

### SUBUNITS AND ANALOGS THEREOF

According to the invention, mutant α and β subunits, mutant TSH heterodimers, TSH analogs, single chain TSH analogs, its fragments or other derivatives thereof, may be used as an immunogen to generate antibodies which immunospecifically bind such an immunogen. Such antibodies include but are not limited to polyclonal, monoclonal, chimeric, single chain, Fab fragments, and an Fab expression library. In a specific embodiment, antibodies to a mutant TSH are produced. In another embodiment, antibodies to a domain of a mutant α or β subunits are produced.

Various procedures known in the art may be used for the production of polyclonal antibodies to mutant α and β subunits, mutant TSH heterodimers, TSH analogs, single chain TSH analogs, its fragments or other derivatives thereof. For the production of antibody, various host animals can be immunized by injection with the subunits, heterodimer, single chain analog, and derivatives thereof, including but not limited to rabbits, mice, rats, etc. Various adjuvants may be used to increase the immunological response, depending on the host species, and including but not limited to Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (bacille Calmette-Guerin) and corynebacterium parvum.

For preparation of monoclonal antibodies directed toward mutant α and β subunits, mutant TSH heterodimers, TSH analogs, single chain TSH analogs, its fragments or other derivatives thereof, any technique which provides for the production of antibody molecules by continuous cell lines in culture may be used. For example, the hybridoma technique originally developed by Kohler and Milstein (1975, Nature 256:495-497), as well as the trioma technique, the human B-cell hybridoma technique (Kozbor et al., 1983, Immunology Today 4:72), and the EBV-hybridoma technique to produce human monoclonal antibodies (Cole et al., 1985, in Monoclonal Antibodies and Cancer Therapy, Alan R. Liss, Inc., pp. 77-96). In an additional embodiment of the invention, monoclonal antibodies can be produced in germ-free animals utilizing recent technology (PCT/US90/02545). According to the invention, human antibodies may be used and can be obtained by using human hybridomas (Cote et al., 1983, Proc. Natl. Acad. Sci. U.S.A. 80:2026-2030) or by transforming human B cells with EBV virus *in vitro* (Cole et al., 1985, in Monoclonal Antibodies and Cancer Therapy, Alan R. Liss, pp. 77-96). In fact, according to the invention, techniques developed for the production of "chimeric antibodies" (Morrison et al., 1984, Proc. Natl. Acad. Sci. U.S.A. 81:6851-6855; Neuberger et al., 1984, Nature 312:604-608; Takeda et al., 1985, Nature 314:452-454) by splicing the genes from a mouse antibody molecule specific for the epitope together with genes from a human antibody molecule of appropriate biological activity can be used; such antibodies are within the scope of this invention.

According to the invention, techniques described for the production of single chain antibodies (U.S. Patent 4,946,778) can be adapted to produce specific single chain antibodies against TSH subunits, heterodimers, single chain analogs, or fragments or derivatives thereof. An additional embodiment of the invention utilizes the techniques described for the construction of Fab expression libraries (Huse et al., 1989, Science 246:1275-1281) to allow rapid and easy identification of monoclonal Fab fragments with the desired specificity.

Antibody fragments which contain the idiotype of the molecule can be generated by known techniques. For example, such fragments include but are not limited to: the F(ab')₂ fragment which can be produced by pepsin digestion of the antibody molecule; the Fab' fragments which can be generated by reducing the disulfide bridges of the F(ab')₂ fragment, the Fab fragments which can be generated by treating the antibody molecule with papain and a reducing agent, and Fv fragments.

In the production of antibodies, screening for the desired antibody can be accomplished by techniques known in the art, *e.g.* ELISA (enzyme-linked immunosorbent assay). For example, to select antibodies which recognize a specific domain of a mutant subunit, one may assay generated hybridomas for a product which binds to a fragment of a mutant subunit containing such domain. For selection of an antibody that specifically binds a mutant subunit, mutant TSH or a single chain analog but which does not specifically bind wild type TSH, one can select on the basis of positive binding to the mutant and a lack of binding to the wild type protein. Antibodies specific to a domain of a mutant subunit, mutant TSH or a single chain analog are also provided.

The foregoing antibodies can be used in methods known in the art relating to the localization and activity of the mutant subunits, mutant TSH or single chain analogs of the invention, *e*.*g*., for imaging these proteins, measuring levels thereof in appropriate physiological samples, in diagnostic methods, etc.

### 5.8 ANALYSIS OF MUTANT TSH SUBUNITS

Described herein are methods for determining the structure of mutant TSH subunits, mutant heterodimers and TSH analogs, and for analyzing the in vitro activities and in vivo biological functions of the foregoing.

Once a mutant α or TSH β subunit is identified, it may be isolated and purified by standard methods including chromatography (*e*.*g*., ion exchange, affinity, and sizing column chromatography), centrifugation, differential solubility, or by any other standard technique for the purification of proteins. The functional properties may be evaluated using any suitable assay (including immunoassays as described *infra*).

Alternatively, once a mutant α subunit and/or TSH β subunit produced by a recombinant host cell is identified, the amino acid sequence of the subunit(s) can be determined by standard techniques for protein sequencing, e.g., with an automated amino acid sequencer.

The mutant subunit sequence can be characterized by a hydrophilicity analysis (Hopp, T. and Woods, K., 1981, Proc. Natl. Acad. Sci. U.S.A. 78:3824). A hydrophilicity profile can be used to identify the hydrophobic and hydrophilic regions of the subunit and the corresponding regions of the gene sequence which encode such regions.

Secondary structural analysis (Chou, P. and Fasman, G., 1974, Biochemistry 13:222) can also be done, to identify regions of the subunit that assume specific secondary structures.

Other methods of structural analysis can also be employed. These include but are not limited to X-ray crystallography (Engstom, A., 1974, Biochem. Exp. Biol. 11:7-13) and computer modeling (Fletterick, R. and Zoller, M. (eds.), 1986, Computer Graphics and Molecular Modeling, in Current Communications in Molecular Biology, Cold Spring Harbor Laboratory, Cold Spring Harbor, New York). Structure prediction, analysis of crystallographic data, sequence alignment, as well as homology modelling, can also be accomplished using computer software programs available in the art, such as BLAST, CHARMm release 21.2 for the Convex, and QUANTA v.3.3, (Molecular Simulations, Inc., York, United Kingdom).

The functional activity of mutant α subunits, mutant β subunits, mutant TSH heterodimers, TSH analogs, single chain analogs, derivatives and fragments thereof can be assayed by various methods known in the art.

For example, where one is assaying for the ability of a mutant subunit or mutant TSH to bind or compete with wild-type TSH or its subunits for binding to an antibody, various immunoassays known in the art can be used, including but not limited to competitive and non-competitive assay systems using techniques such as radioimmunoassays, ELISA (enzyme linked immunosorbent assay), "sandwich" immunoassays, immunoradiometric assays, gel diffusion precipitin reactions, immunodiffusion assays, *in situ* immunoassays (using colloidal gold, enzyme or radioisotope labels, for example), western blots, precipitation reactions, agglutination assays (*e*.*g*., gel agglutination assays, hemagglutination assays), complement fixation assays, immunofluorescence assays, protein A assays, and immunoelectrophoresis assays, etc. Antibody binding can be detected by detecting a label on the primary antibody. Alternatively, the primary antibody is detected by detecting binding of a secondary antibody or reagent to the primary antibody, particularly where the secondary antibody is labelled. Many means are known in the art for detecting binding in an immunoassay and are within the scope of the present invention.

The binding of mutant α subunits, mutant β subunits, mutant TSH heterodimers, TSH analogs, single chain analogs, derivatives and fragments thereof, to the thyroid stimulating hormone receptor (TSHR) can be determined by methods well-known in the art, such as but not limited to in vitro assays based on displacement from the TSHR of a radiolabelled TSH of another species, such as bovine TSH, for example, but not limited to, as described by Szkudlinski et al. (1993, Endocrinol. 133:1490-1503). The bioactivity of mutant TSH heterodimers, TSH analogs, single chain analogs, derivatives and fragments thereof, can also be measured, for example, by assays based on cyclic AMP stimulation in cells expressing TSHR, for example but not limited to assays described in Section 6.2.3 *infra* and by Grossmann et al. (1995, Mol. Endocrinol. 9:948-958); and stimulation of thymidine uptake in thyroid cells, for example but not limited to as described by Szkudlinski et al. (1993, Endocrinol. 133:1490-1503).

In vivo bioactivity can be determined by physiological correlates of TSHR binding in animal models, such as measurements of T4 secretion in mice after injection of the mutant TSH heterodimer, TSH analog, or single chain analog, *e.g.* as described by East-Palmer et al. (1995, Thyroid 5:55-59) and in Section 6.2, *supra.* For example, wild type TSH and mutant TSH are injected intraperitoneally into male albino Swiss Crl:CF-1 mice with previously suppressed endogenous TSH by administration of 3 µg/ml T₃ in drinking water for 6 days. Blood samples are collected 6 h later from orbital sinus and the serum T₄ and TSH levels are measured by respective chemiluminescence assays (Nichols institute).

The half-life of a protein is a measurement of protein stability and indicates the time necessary for a one-half reduction in the concentration of the protein. The half life of a mutant TSH can be determined by any method for measuring TSH levels in samples from a subject over a period of time, for example but not limited to, immunoassays using anti-TSH antibodies to measure the mutant TSH levels in samples taken over a period of time after administration of the mutant TSH or detection of radiolabelled mutant TSH in samples taken from a subject after administration of the radiolabelled mutant TSH.

Other methods will be known to the skilled artisan and are within the scope of the invention.

### 5.9 DIAGNOSTIC AND THERAPEUTIC USES

The invention provides for treatment or prevention of various diseases and disorders by administration of therapeutic compound (termed herein "Therapeutic") of the invention. Such Therapeutics include TSH heterodimers having a mutant α subunit having at least an amino acid substitution at position 22 of the α subunit as depicted in Figure 1 (SEQ ID NO:1) and either a mutant or wild type β subunit; TSH heterodimers having a mutant α subunit, preferably with one or more amino acid substitutions in or near the L1 loop (amino acids 8-30 as depicted in Figure 1 (SEQ ID NO:1)) and a mutant β subunit, preferably with one or more amino acid substitutions in or near the L3 loop (amino acids 52-87 as depicted in Figure 2 (SEQ ID NO:2)) and covalently bound to the CTEP of the β subunit of hCG; TSH heterodimers having a mutant α subunit, preferably with one or more amino acid substitutions in or near the L1 loop, and a mutant β subunit, preferably with one or more amino acid substitutions in or near the L3 loop, where the mutant α subunit and the mutant β subunit are covalently bound to form a single chain analog, including a TSH heterodimer where the mutant α subunit and the mutant β subunit and the CTEP of the β subunit of hCG are covalently bound in a single chain analog, other derivatives, analogs and fragments thereof (e.g. as described hereinabove) and nucleic acids encoding the mutant TSH heterodimers of the invention, and derivatives, analogs, and fragments thereof.

The subject to which the Therapeutic is administered is preferably an animal, including but not limited to animals such as cows, pigs, horses, chickens, cats, dogs, etc., and is preferably a mammal. In a preferred embodiment, the subject is a human. Generally, administration of products of a species origin that is the same species as that of the subject is preferred. Thus, in a preferred embodiment, a human mutant and/or modified TSH heterodimer, derivative or analog, or nucleic acid, is therapeutically or prophylactically or diagnostically administered to a human patient.

In a preferred aspect, the Therapeutic of the invention is substantially purified.

A number of disorders which manifest as hypothyroidism can be treated by the methods of the invention. Disorders in which TSH is absent or decreased relative to normal or desired levels are treated or prevented by administration of a mutant TSH heterodimer or TSH analog of the invention. Disorders in which TSH receptor is absent or decreased relative to normal levels or unresponsive or less responsive than normal TSHR to wild type TSH, can also be treated by administration of a mutant TSH heterodimer or TSH analog. Constitutively active TSHR can lead to hyperthyroidism, and it is contemplated that mutant TSH heterodimers and TSH analogs can be used as antagonists.

In specific embodiments, mutant TSH heterodimers or TSH analogs that are capable of stimulating differentiated thyroid functions are administered therapeutically, including prophylactically. Diseases and disorders that can be treated or prevented include but are not limited to hypothyroidism, hyperthyroidism, thyroid development, thyroid cancer, benign goiters, enlarged thyroid, protection of thyroid cells from apoptosis, etc.

The absence of decreased level in TSH protein or function, or TSHR protein and function can be readily detected, *e*.*g*., by obtaining a patient tissue sample (*e*.*g*., from biopsy tissue) and assaying it *in vitro* for RNA or protein levels, structure and/or activity of the expressed RNA or protein of TSH or TSHR. Many methods standard in the art can be thus employed, including but not limited to immunoassays to detect and/or visualize TSH or TSHR protein (*e*.*g*., Western blot, immunoprecipitation followed by sodium dodecyl sulfate polyacrylamide gel electrophoresis, immunocytochemistry, etc.) and/or hybridization assays to detect TSH or TSHR expression by detecting and/or visualizing TSH or TSHR mRNA (*e*.*g*., Northern assays, dot blots, *in situ* hybridization, etc.), etc.

In specific embodiments, Therapeutics of the invention are used to treat cancer of the thyroid. The mutant TSH heterodimers and analogs are useful in the stimulation of thyroidal and metastatic tissue prior to therapeutic ablation with radioactive iodine. For example, the mutant TSH heterodimers of the invention can be administered to a patient suffering from thyroidal cancer prior to administration of radiolabelled iodine for radioablation. The Therapeutics of the invention can also be used to stimulate iodine uptake by benign multinodular goiters prior to radioablation for treatment of the multinodular goiters, or to stimulate iodine uptake by thyroid tissue prior to radioablation for treatment of enlarged thyroid.

Specifically, the radioablation therapy is carried out by administering the Therapeutic of the invention, preferably administering the Therapeutic intramuscularly, in a regimen of one to three doses, for example but not limited to, one dose per day for two days, or one dose on the first, fourth and seventh days of a seven day regimen. The dosage is any appropriate dose, as described in Section 5.10 *infra,* for example but not limited to a dose of approximately 10 µg to 1 mg, preferably a dose of approximately 10 µg to 100 µg. One day after the last dose of the regimen, radiolabelled iodine, preferably ¹³¹I, is administered to the subject in an amount sufficient to treat the cancer, noncancerous goiter or enlarged thyroid. The amount of radiolabelled iodine to be administered will depend upon the type and severity of the disease. In general, 30 to 300 mCi of ¹³¹I is administered to treat thyroid carcinoma.

In other specific embodiments, the mutant TSH heterodimers of the invention can be used for targetted delivery of therapeutics to the thyroid or to thyroid cancer cells, *e*.*g*. for targetted delivery of nucleic acids for gene therapy (for example targetted delivery of tumor suppressor genes to thyroid cancer cells) or for targetted delivery of toxins such as, but not limited to, ricin, diptheria toxin, etc.

The invention further provides methods of diagnosis, prognosis, screening for thyroid cancer, preferably thyroid carcinoma, and of monitoring treatment of thyroid cancer, for example, by administration of the TSH heterodimers of the invention. In specific embodiments, Therapeutics of the invention are administered to a subject to stimulate uptake of iodine (preferably radiolabelled iodine such as, but not limited to, ¹³¹I or ¹²⁵I) by thyroid cells (including thyroid cancer cells) and/or to stimulate secretion of thyroglobulin from thyroid cells (including thyroid cancer cells). Subsequent to administration of the Therapeutic, radiolabelled iodine can be administered to the patient, and then the presence and localization of the radiolabelled iodine (*i*.*e*. the thyroid cells) can be detected in the subject (for example, but not by way of limitation, by whole body scanning) and/or the levels of thyroglobulin can be measured or detected in the subject, wherein increased levels of radioactive iodine uptake or increased levels of thyroglobulin secretion, as compared to levels in a subject not suffering from a thyroid cancer or disease or to a standard level, indicates that the subject has thyroid cancer. Certain subjects may have undergone thyroidectomy or thyroid tissue ablation therapy and have little or no residual thyroid tissue. In these subjects, or any other subject lacking noncancerous thyroid cells, detection of any iodine uptake or thyroglobulin secretion (above any residual levels remaining after the thyroidectomy or ablation therapy or after the loss of thyroid tissue for any other reason) indicates the presence of thyroid cancer cells. The localization of the incorporated radiolabelled iodine in the subject can be used to detect the spread or metastasis of the disease or malignancy. Additionally, the diagnostic methods of the invention can be used to monitor treatment of thyroid cancer by measuring the change in radiolabelled iodine or thyroglobulin levels in response to a course of treatment or by detecting regression or growth of thyroid tumor or metastasis.

Specifically, the diagnostic methods are carried out by administering the Therapeutic of the invention, preferably intramuscularly, in a regimen of one to three doses, for example but not limited to, one dose per day for two days, or one dose on the first, fourth and seventh days of a seven day regimen. The dosage is any appropriate dose, as described in Section 5.10 *infra,* for example but not limited to a dose of approximately 10 µg to 1 mg, preferably a dose of approximately 10 µg to 100 µg. One day after the last dose of the regimen, radiolabelled iodine, preferably ¹³¹I, is administered to the subject in an amount sufficient for the detection of thyroid cells (including cancer cells), in general, 1-5 mCi of ¹³¹I is administered to diagnose thyroid carcinoma. Two days after administration of the radiolabelled iodine, the uptake of radiolabelled iodine in the patient is detected and/or localized in the patient, for example but not limited to, by whole body radioiodine scanning. Alternatively, in cases where all or most of the thyroid tissue has been removed (*e*.*g*. in patients with prior thyroidectomy or thyroid tissue ablation therapy), levels of thyroglobulin can be measured from 2 to 7 days after administration of the last dose of the Therapeutic of the invention. Thyroglobulin can be measured by any method well known in the art, including use of a immunoradiometric assay specific for thyroglobulin, which assay is well known in the art.

The mutant TSH heterodimers of the invention can also be used in TSH binding inhibition assays for TSH receptor autoantibodies, e.g. as described in Kakinuma et al. (1997, J. Clin. Endo. Met. 82:2129-2134). Antibodies against the TSH receptor are involved in certain thyroid diseases, such as but not limited to Graves' disease and Hashimoto's thyroiditis; thus, these binding inhibition assays can be used as a diagnostic for diseases of the thyroid such as Graves' disease and Hashimoto's thyroiditis. Briefly, cells or membrane containing the TSH receptor are contacted with the sample to be tested for TSHR antibodies and with radiolabelled mutant TSH of the invention, inhibition of the binding of the radiolabelled mutant TSH of the invention relative to binding to cells or membranes contacted with the radiolabelled mutant TSH but not with the sample to be tested indicates that the sample to be tested has antibodies which bind to the TSH receptor. The binding inhibition assay using the mutant TSH heterodimers of the invention, which have a greater bioactivity than the wild type TSH, has greater sensitivity for the anti-TSH receptor antibodies than does a binding inhibition assay using wild type TSH.

Accordingly, an embodiment of the invention provides methods of diagnosing or screening for a disease or disorder characterized by the presence of antibodies to the TSHR, preferably Graves' disease, comprising contacting cultured cells or isolated membrane containing TSH receptors with a sample putatively containing the antibodies from a subject and with a diagnostically effective amount of a radiolabelled mutant TSH heterodimer of the invention; measuring the binding of the radiolabelled mutant TSH to the cultured cells or isolated membrane, wherein a decrease in the binding of the radiolabelled TSH relative to the binding in the absence of said sample or in the presence of an analogous sample not having said disease or disorder, indicates the presence of said disease or disorder.

The mutant heterodimers and analogs may also be used in diagnostic methods to detect suppressed, but functional thyroid tissue in patients with autonomous hyperfunctioning thyroid nodules or exogenous thyroid hormone therapy. The mutant TSH heterodimers and TSH analogs may have other applications such as but not limited to those related to the diagnosis of central and combined primary and central hypothyroidism, hemiatrophy of the thyroid, and resistance to TSH action.

### 5.10 PHARMACEUTICAL COMPOSITIONS

The invention provides methods of diagnosis and methods of treatment by administration to a subject of an effective amount of a Therapeutic of the invention. In a preferred aspect, the Therapeutic is substantially purified. The subject is preferably an animal, including but not limited to animals such as cows, pigs, horses, chickens, cats, dogs, etc., and is preferably a mammal, and most preferably human. In a specific embodiment, a non-human mammal is the subject.

The mutant TSH heterodimers and TSH analogs of the invention are preferably tested *in vitro,* and then *in vivo* for the desired, prior to use in humans. In various specific embodiments, *in vitro* assays can be carried out with representative cells of cell types (e.g., thyroid cells) involved in a patient's disorder, to determine if a mutant TSH heterodimer or TSH analog has a desired effect upon such cell types, *e*.*g*. as described in Section 5.8, *supra.*

Compounds for use in therapy can be tested in suitable animal model systems prior to testing in humans, including but not limited to rats, mice, chicken, cows, monkeys, rabbits, etc. For *in vivo* testing, prior to administration to humans, any animal model system known in the art may be used.

Various delivery systems are known and can be used to administer a mutant TSH heterodimer or TSH analog of the invention, e.g., encapsulation in liposomes, microparticles, microcapsules, recombinant cells capable of expressing the mutant TSH heterodimer or TSH analog, receptor-mediated endocytosis (see, e.g., Wu and Wu, 1987, J. BioL Chem. 262:4429-4432), etc. Methods of introduction include but are not limited to intradermal, intramuscular, intraperitoneal, intravenous, subcutaneous, intranasal, epidural, and oral routes. The compounds may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local. In addition, it may be desirable to introduce the pharmaceutical compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In a specific embodiment, it may be desirable to administer the pharmaceutical compositions of the invention locally to the area in need of treatment; this may be achieved by, for example, and not by way of limitation, local infusion during surgery, by means of a catheter, by means of a suppository, or by means of an implant, said implant being of a porous, non-porous, or gelatinous material, including membranes, such as sialastic membranes, or fibers.

In another embodiment, the mutant TSH heterodimer or TSH analog can be delivered in a vesicle, in particular a liposome (see Langer, Science 249:1527-1533 (1990); Treat et al., in Liposomes in the Therapy of Infectious Disease and Cancer, Lopez-Berestein and Fidler (eds.), Liss, New York, pp. 353-365 (1989); Lopez-Berestein, ibid., pp. 317-327; see generally ibid.)

In yet another embodiment, the mutant TSH heterodimer or TSH analog can be delivered in a controlled release system. In one embodiment, a pump may be used (see Langer, supra; Sefton, CRC Crit. Ref. Biomed. Eng. 14:201 (1987); Buchwald et al., Surgery 88:507 (1980); Saudek et al., N. EngL J. Med. 321:574 (1989)). In another embodiment, polymeric materials can be used (see Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, J. Macromol. Sci. Rev. Macromol. Chem. 23:61 (1983); see also Levy et al., Science 228:190 (1985); During et al., Ann. Neurol. 25:351 (1989); Howard et al., J. Neurosurg. 71:105 (1989)). In yet another embodiment, a controlled release system can be placed in proximity of the therapeutic target, thus requiring only a fraction of the systemic dose (see, e.g., Goodson, in Medical Applications of Controlled Release, supra, vol. 2, pp. 115-138 (1984)).

Other controlled release systems are discussed in the review by Langer (Science 249:1527-1533 (1990)).

In a specific embodiment, a nucleic acid encoding mutant TSH heterodimer or TSH analog can be administered *in vivo* to promote expression of its encoded protein, by constructing it as part of an appropriate nucleic acid expression vector and administering it so that it becomes intracellular, *e*.*g*., by use of a retroviral vector (see U.S. Patent No. 4,980,286), or by direct injection, or by use of microparticle bombardment (*e*.*g*., a gene gun; Biolistic, Dupont), or coating with lipids or cell-surface receptors or transfecting agents, or by administering it in linkage to a homeobox-like peptide which is known to enter the nucleus (see *e*.*g*., Joliot et al., 1991, Proc. Natl. Acad. Sci. USA 88:1864-1868), etc. Alternatively, a nucleic acid molecule encoding a mutant TSH heterodimer or TSH analog can be introduced intracellularly and incorporated within host cell DNA for expression, by homologous recombination.

The present invention also provides pharmaceutical compositions. Such compositions comprise a therapeutically effective amount of a mutant TSH heterodimer or TSH analog, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the mutant TSH heterodimer or TSH analog, preferably in purified form, together with a suitable amount of carrier so as to provide the form for proper administration to the patient. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The mutant TSH heterodimers or TSH analogs of the invention can be formulated as neutral or salt forms. Pharmaceutically acceptable salts include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylamino ethanol, histidine, procaine, etc.

The amount of the mutant TSH heterodimer or TSH analog of the invention which will be effective in the treatment of a particular disorder or condition will depend on the nature of the disorder or condition, and can be determined by standard clinical techniques. In addition, *in vitro* assays and animal models may optionally be employed to help identify optimal dosage ranges. The precise dose to be employed in the formulation will also depend on the route of administration, and the seriousness of the disease or disorder, and should be decided according to the judgment of the practitioner and each patient's circumstances.

In specific embodiments, the Therapeutics of the invention are administered intramuscularly. Suitable dosage ranges for the intramuscular administration are generally about 10 µg to 1 mg per dose, preferably about 10 µg to 100 µg per dose. Generally, for diagnostic and therapeutic methods in which the mutant TSH heterodimers are administered to stimulate iodine uptake, the mutant TSH heterodimers can be administered in a regimen of 1-3 injections. In one embodiment, the Therapeutic is administered in two doses, where the second dose is administered 24 hours after the first dose; in another embodiment, the Therapeutic is administered in three doses, with one dose being administered on days 1, 4 and 7 of a 7 day regimen.

Effective doses may be extrapolated from dose-response curves derived from *in vitro* or animal model test systems.

Suppositories generally contain active ingredient in the range of 0.5% to 10% by weight; oral formulations preferably contain 10% to 95% active ingredient.

The invention also provides a pack or kit for therapeutic or diagnostic use comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or diagnostic products, which notice reflects approval by the agency of manufacture, use or sale for human administration.

### 6. EXAMPLES

Two examples of novel mutant TSH heterodimers are provided hereinbelow. The data shows that the mutant TSH heterodimers have higher bioactivity than wild type TSH.

### 6.1 MATERIALS

Restriction enzymes, DNA markers and other molecular biological reagents were purchased from either Gibco BRL (Gaithersburg, MD) or from Boehringer-Mannheim (Indianapolis, IN). Cell culture media, fetal bovine serum, and LIPOFECTAMINE were purchased from New England Biolabs (Beverly, MA). The full-length human α cDNA (840 bp) subcloned into BamHI/XhoI sites of the pcDNA I/Neo vector (Invitrogen, San Diego, CA) and hCG-β gene were obtained from T.H. Ji (University of Wyoming, Laramie, WY). The hTSH-β minigene without the first intron, with the nontranslated first exon and authentic translation initiation site was constructed by the inventors. Recombinant human TSH standard was from Genzyme (Framingham, MA). The Chinese Hamster Ovary (CHO) cells with stably expressed hTSH receptor (CHO-hTSHR clone JP09 and clone JP26) were provided by G. Vassart (University of Brussels, Brussels, Belgium). ¹²⁵I cAMP, ¹²⁵I-human TSH, and ¹²⁵I-bovine TSH radiolabelled to a specific activity of 40-60 µCi/µg were obtained from Hazleton Biologicals (Vienna, VA).

### 6.2 METHODS

### 6.2.1 SITE-DIRECTED MUTAGENESIS

Site-directed mutagenesis of the human α-cDNA was accomplished by the PCR-based megaprimer method (Sarkar et al., 1990, Biotechniques, 8:404-407). Amplification was optimized using Vent^{®} DNA Polymerase (New England Biolabs). After digestion with BamHI and Xhol, PCR product was ligated into pcDNA I/Neo (Invitrogen) with the BamHI/XhoI fragment excised. MC1061/p3 *Escherichia coli* cells were transformed using Ultracomp *E coli* Transformation Kit (Invitrogen). The QIAprep 8 Plasmid Kit (Qiagen) was used for multiple plasmid DNA preparations. Qiagen Mega and Maxi Purification Protocols were used to purify larger quantities of plasmids containing α-cDNA with single or multiple mutations as a template for further mutagenesis. Mutations were confirmed by doublestranded sequencing using Sanger's dideoxynucteotide chain termination procedure. The construction of the mutant TSH β submit fusion with the CTEP is described in Joshi et al. (1995, Endocrinology, 136:3839-3848).

### 6.2.2 EXPRESSION OF RECOMBINANT HORMONES

CHO-K1 Cells (ATCC, Rockville, MD) were maintained in HAM's F-12 medium with glutamine and 10% FBS, penicillin (50 units/ml), and streptomycin (50 µg/ml). Plates of cells (100-mm culture dishes) were cotransfected with wild type or mutant α-cDNA in the pcDNA I/NEO and mutant hTSHβ minigene inserted into the p(LB)CMV vector, using a LIPOFECTAMINE Reagent (Gibco BRL). After 24 h, the transfected cells were transferred to CHO-serum free sodium (CHO-SFM-II, Gibco BRL). The culture media including control medium from mock transfections using the expression plasmids without gene inserts were harvested 72 h after transfection, concentrated, and centrifuged; the aliquots were stored at -20°C and thawed only once before each assay. Wild type and mutant hTSH were measured and verified using various activity essays and immunoassays.

### 6.2.3 cAMP STIMULATION IN MAMMALIAN

### CELLS EXPRESSING THE HUMAN TSH RECEPTOR

CHO-K1 cells stably transfected with hTSH receptor cDNA (JP09 or JP26) were grown and incubated with serial dilutions of wild type and mutant TSH as described. cAMP released into the medium was determined by radioimmunoassay. The equivalent amounts of total media protein were used as the mock control and the hTSH-containing samples from transfected cells.

### 6.2.4 IN VIVO TSH BIOACTIVITY ASSAYS

The thyrotropic activity of the hTSH was assessed by its ability to induce cAMP production in CHO cells expressing hTSH receptors (clones JP09 and JP26) and in FRLT-5 cells expressing endogenous rat TSH receptor. FRTL-5 cells were also used to test the hTSH-induced cell growth. To that end, CHO cells stably expressing the hTSH receptor (JP09 or JP26), were grown to confluence in supplemented HAM's F12 medium in 96-well tissue culture plates. Subsequently, cells were incubated either in salt-free conditions or with physiologic (0.9%) NaCl concentration for 2 hours at 37°C, 5% CO₂ with serial dilutions of wild type and mutant hTSH as well as control medium from mock transfections. The amount of cAMP released into the medium was determined by radioimmunoassay. In vivo activity of the hTSH analogs was tested by determination of total thyroxine (total T₄) levels after intraperitoneal injections into T₃-suppressed mice as described by Szkudlinski et al., in Nat. Biotechnol. 14:1257 (1996).

### 6.3 RESULTS

The results presented in Figures 4-8 support the conclusion that TSH heterodimers mutated in accordance with the invention exhibited enhanced biological activity when compared with the corresponding wild type TSH. More particularly, the results indicated that single or multiple mutations within the TSH subunits in the above-described procedures could be incorporated into the heterodimers having enhanced activity in vitro and in vivo. This was true for several different mutations and combinations thereof, and so illustrates the principal underlying the present invention.

In one example illustrating that mutation in the αL1 loop of the common human α subunit increased hormone activity there was created a mutant wherein a the glycine residue ordinarily present at position 22 of the α-subunit was substituted by an arginine residue (αG22R). A mutant TSH heterodimer comprising this mutation in combination with a wild type β subunit was produced by transiently coexpressing the individual subunits in CHO-K1 cells. The resulting mutant heterodimer was then tested in a bioactivity assay using CHO-JP26 cells that express the TSH receptor. As indicated by the results presented in Figure 4, the mutant hormone bound TSHR and induced a higher level of cyclic AMP production than did the wild type TSH.

The plasma half life and stability of mutant TSH superactive heterodimers, wild type or mutant common α subunits comprising four mutations, Q13K+E14K+P16K+Q20K, i.e., α4K, was increased by coexpressing the α4K subunit and the wild type human TSH β subunit or human TSH β subunit fusion with CTEP of hCG (β-CTEP) in CHO-K1 cells. Wild type and mutant TSH heterodimers were quantitated using a chemiluminescence immunoassay (Nichols Institute). The results are shown in Table 1 (100% expression is 47 ng wild type TSH per ml).

**TABLE 1**

| | Expression | SEM |
|---|---|---|
| | (%WT) | |
| hTSH Wild Type | 100 | 6 |
| hTSH-α4K | 26 | 5 |
| hTSH-α4K+CTEP | 20 | 3 |

The presence of CTEP did not reduce expression of the heterodimer comprising α4K and the β-CTEP fusion protein in comparison with the heterodimer comprising α4K and wild type TSHβ.

The ability of wild type and mutant TSH heterodimers to bind the TSHR was assessed by the stimulation of cyclic AMP production in CHO-JP09 that stably express a transfected TSHR. As indicated by the results presented in Figure 5, the α4K/β-CTEP heterodimer showed 200 fold increase of potency and 1.5 fold increase in Vmax compared to wild type TSH. It was surprising that the inclusion of CTEP, which is expected to prolong the in vivo half life of the α4K/β-CTEP heterodimer, also increased its in vitro activity a further 3-4 fold over that of a α4K/wild type β heterodimer.

The results show that mutations which increase the bioactivity of a mutant TSH can be advantageously combined with a mutation or modification that prolongs circulatory half life to create mutant hormones that display superior in vitro and in vivo characteristics.

In other examples, mutations in the β hairpin L3 loop of the common human α-subunit also increased hormone activity. One of the mutations was a substitution of the alanine residue at position 81 with a lysine residue (αA81K). The other mutation was a substitution of the asparagine residue at position 66 with a lysine residue (αN66K). Each of the mutant human α-subunits was transiently expressed in CHO-K1 cells in combination with wild type human TSH-β subunits to produce mutant TSH heterodimers. Each of these mutant TSH heterodimers was tested in a bioactivity assay using CHO-JP09 cells that expressed the human TSH receptor. Results from these procedures indicated that both mutant hormones stimulated higher levels of cAMP production than did the wild type hormone. Substitution of alanine 81 to lysine (αA81K) in the α-subunit represents the first demonstration of introduction of a lysine residue, which is not present in other homologous sequences, into a β hairpin loop.

In yet another example, a mutation near the β hairpin L1 loop of the human TSH β subunit increased the hormone activity of a heterodimer that included this mutant subunit. The mutation was a substitution of the glutamate residue at position 6 with an asparagine residue (βE6N) which eliminates a negatively charged residue in the periphery of the β hairpin L1 loop. The mutant human TSH-β subunit was transiently expressed in CHO-K1 cells in combination with a wild type human common α-subunit to produce a mutant TSH heterodimer. The mutant TSH heterodimer was then tested in a bioactivity assay using CHO-JP09 cells that expressed the TSH receptor. Again, results indicated that this mutant TSH hormone bound the receptor and induced higher levels of cAMP production than did the wild type TSH.

The results presented in Figures 6 and 7 further confirm that mutations in the βL1 loop can be used to produce mutant heterodimers advantageously possessing enhanced biological activity when tested using in vitro assays. More particularly, the results presented in Figure 6 indicated that the individual mutants βF1R, βE6N and βA17R could be combined with wild type α subunit to form mutant heterodimers possessing enhanced hormone activity. Figure 7 shows that β subunits included the combination of either βF1R and βE6N, or βF1R, βE6N and βA17R also possessed enhanced hormone activity.

In accordance with the in vitro findings described above, the mutant hTSH analogs exhibited parallel increases in their in vivo activities. Indeed, in one demonstration of the in vivo activity of TSH mutants prepared in accordance with the procedure described above there was created a TSH-β subunit (β3R) having three point mutations: βI58R, βE63R and βL69R. The above-described α4K mutant α-subunit and the β3R mutant mutant β-subunit were coexpressed intracellularly, collected from conditioned medium and the resulting heterodimer tested for biological activity measurable as total T₄. Mutagenesis did not significantly influence the clearance of the analogs from the circulation. The results shown in Figure 8A indicated that two different mutant heterodimers exhibited dramatically enhanced bioactivity in vivo. The results shown in Figure 8B indicated that the magnitude of the increased bioactivity of the α4K/β3R heterodimer relative to the wild type control was at least 100 fold. Moreover, these results confirmed that combinations of mutant TSH subunits could dramatically enhance hormone activity in vivo. In light of these results, it is reasonably expected that TSH mutants and analogs as described herein will be superior to conventional recombinant hTSH in the diagnostic management of thyroid cancer.

The results presented above confirm that mutation of the TSH subunits in accordance with the teaching provided herein advantageously could be used to make and use TSH mutants having enhanced biological activities.

The results presented herein further indicate that the peripheral regions of the αL1 and βL3 loops of hTSH represented "modification-permissive" domains, which can be engineered for higher receptor binding and activity. The location of these modification-permissive domains creates a bivalent ligand in which such symmetry of binding interfaces is a result of head to tail dimerization of homodimers or heterodimers and believed to mediate ligand-induced receptor dimerization. Although functionally relevant receptor dimerization has not been described for any G protein-coupled receptor, a putative interaction site has been localized to the sixth transmembrane domain of adrenergic receptors, and the corresponding region is a "hot spot" for constitutively activating mutations of the TSH receptor.

Thus, using a rational strategy based on evolutionary considerations and homology comparisons, by testing less than 20 mutants, we have identified the α4K/β3R hTSH analog having only seven mutations out of a total of 204 amino acids of the TSH molecule and having up to a 50,000-fold increase in binding affinity and up to 1,300-fold increase in hormone potency. While not wishing to be bound by any particular theory of operation, it is possible that modulation of peripheral loops during glyco-protein hormone evolution may have modified hormone function at various phylogenetic stages. The synergy of the two engineered loop regions in receptor binding suggests that combining modifications in such spatially distant domains, which were optimized at different stages of hormone evolution, may provide a universal strategy for engineering human protein analogs. Recombinant analogs of the type described herein have a combination of basic residues that are not present in any known natural hormone at any evolutionary stage, and exceed receptor binding affinity or activity of TSH from any species.

It will be appreciated that certain variations to this invention may suggest themselves to those skilled in the art. The foregoing detailed description is to be clearly understood as given by way of illustration, the spirit and scope of this invention being interpreted upon reference to the appended claims.

### FURTHER ASPECTS

Further aspects and embodiments of the invention are now set out in the following numbered Paragraphs; it is to be understood that the invention encompasses these aspects:
Paragraph 1. A mutant α subunit comprising an amino acid substitution at position 22 of the amino acid sequence of the α subunit as depicted in Figure 1 (SEQ ID NO: 1).
Paragraph 2. The mutant α subunit of Paragraph 1 wherein the amino acid substitution at position 22 is arginine.
Paragraph 3. The mutant α subunit of Paragraph 1 which is purified.
Paragraph 4. The mutant α subunit of Paragraph 1 further comprising one or more amino acid substitutions in amino acid residues selected from among positions 11-21 of the amino acid sequence of the α subunit as depicted in Figure 1 (SEQ ID NO: 1).
Paragraph 5. The mutant α subunit of Paragraph 5 wherein the one or more amino acid substitutions are in amino acid residues selected from among positions 11, 13, 14, 16, 17 and 20 of the amino acid sequence of the α subunit as depicted in Figure 1 (SEQ ID NO: 1).
Paragraph 6. The mutant α subunit of Paragraph 1 further comprising one or more amino acid substitutions in the β hairpin L1 loop of the α subunit.
Paragraph 7. The mutant α subunit of Paragraph 4 or 6 wherein the one or more substitutions are amino acids selected from the group consisting of arginine and lysine.
Paragraph 8. The human α subunit of Paragraph 4 wherein the one or more substitutions are selected from the group consisting of αT11K, αQ13K, αE14K, αP16K, αF17R, and αQ20K.
Paragraph 9. A mutant α subunit in which the only mutation is an amino acid substitution at position 22 of the amino acid sequence of the α subunit as depicted in Figure 1 (SEQ ID NO: 1).
Paragraph 10. The mutant α subunit of Paragraph 9 wherein the substitution at position 22 is arginine.
Paragraph 11. The mutant α subunit of Paragraph 9 which is purified.
Paragraph 12. A mutant TSH heterodimer comprising a mutant α subunit comprising an amino acid substitution at position 22 of the amino acid sequence of the α subunit as depicted in Figure 1 (SEQ ID NO: 1) and a β subunit, wherein the bioactivity of the mutant TSH heterodimer is greater than the bioactivity of the wild type TSH heterodimer.
Paragraph 13. The mutant TSH heterodimer of Paragraph 12 wherein the amino acid substitution at position 22 is arginine.
Paragraph 14. The mutant TSH heterodimer of Paragraph 12 which is purified.
Paragraph 15. The mutant TSH heterodimer of Paragraph 12 in which the β subunit is a human β subunit.
Paragraph 16. The mutant TSH heterodimer of Paragraph 12 further comprising one or more amino acid substitutions in amino acid residues selected from among positions 11-21 of the amino acid sequence of the α subunit as depicted in Figure 1 (SEQ ID NO: 1).
Paragraph 17. The mutant TSH heterodimer of Paragraph 12 wherein the one or more amino acid substitutions are in amino acid residues selected from among positions 11, 13, 14, 16, 17, and 20 of the amino acid sequence of the α subunit.
Paragraph 18. The mutant TSH heterodimer of Paragraph 12 further comprising one or more amino acid substitutions in the β hairpin L1 loop of the α subunit.
Paragraph 19. The mutant TSH heterodimer of Paragraph 16 or 18 wherein the one or more substitutions are amino acids selected from the group consisting of arginine and lysine.
Paragraph 20. The mutant TSH heterodimer of Paragraph 16 wherein the one or more substitutions are selected from the group consisting of αT11K, αQ13K, αE14K, αP 16K, αF17R, and αQ20K.
Paragraph 21. A mutant TSH heterodimer in which the only mutation is an amino acid substitution at position 22 of the amino acid sequence of the α subunit as depicted in Figure 1 (SEQ ID NO: 1).
Paragraph 22. The mutant TSH heterodimer of Paragraph 21 wherein the substitution at position 22 is arginine.
Paragraph 23. The mutant heterodimer of Paragraph 21 which is purified.
Paragraph 24. A mutant TSH heterodimer comprising (a) a TSHβ subunit joined via a peptide at its carboxyl terminus to the amino terminus at the carboxyl terminal extension peptide of human chorionic gonadotropin; and (b) an α subunit, wherein at least the TSH β subunit or the TSH α subunit contains at least one amino acid substitution, and wherein the bioactivity of the mutant TSH heterodimer is greater than the bioactivity of wild type TSH heterodimer.
Paragraph 25. The mutant TSH heterodimer of Paragraph 24 wherein the at least one amino acid substitution is in amino acid residues selected from among positions 11-21 of the amino acid sequence of human α subunit as depicted in Figure 1 (SEQ ID NO: 1).
Paragraph 26. The mutant human TSH heterodimer of Paragraph 24 wherein the at least one amino acid substitution is in amino acid residues selected from among positions 58-69 of the amino acid sequence of TSH β subunit as depicted in Figure 2 (SEQ ID NO: 2).
Paragraph 27. The mutant human TSH heterodimer of Paragraph 26 wherein the at least one amino acid substitution is selected from the group consisting of β158R, βE63R and βL69R.
Paragraph 28. The mutant TSH heterodimer of Paragraph 24 comprising a mutant human α subunit and a mutant human TSHβ subunit, wherein the mutant human α subunit comprises at least one amino acid substitution in amino acid residues selected from among positions 11-22 of the amino acid sequence of human α subunit as depicted in Figure 1 (SEQ ID NO: 1), and wherein the mutant human TSH β subunit comprises at least one amino acid substitution in amino acid residues selected from among positions 58-69 of the amino acid sequence of human TSH β subunit as depicted in Figure 2 (SEQ ID NO: 2).
Paragraph 29. The mutant TSH heterodimer of Paragraph 24 which is a mutant of human TSH heterodimer.
Paragraph 30. A TSH analog comprising an α subunit which is covalently bound to a TSH β subunit, wherein at least one of the subunits comprises at least one amino acid substitution in its amino acid sequence, and wherein the bioactivity of said TSH analog is greater than the bioactivity of a TSH analog comprising a wild-type α subunit covalently bound to a wild-type TSH β subunit.
Paragraph 31. The TSH analog of Paragraph 30 wherein the at least one amino acid substitution is in amino acid residues selected from among positions 11-22 of the amino acid sequence of α subunit as depicted in Figure 1 (SEQ ID NO: 1).
Paragraph 32. The TSH analog of Paragraph 30 wherein the at least one amino acid substitution is in amino acid residues selected from among positions 58-69 of the amino acid sequence of human TSH β subunit as depicted in Figure 2 (SEQ ID NO: 2).
Paragraph 33. The TSH analog of Paragraph 30 in which both the α subunit and the β TSH subunit comprise one or more amino acid substitutions.
Paragraph 34. The TSH analog of Paragraph 33 in which the α subunit has at least one amino acid substitution in amino acid residues selected from among positions 11-22 of the amino acid sequence of human α subunit as depicted in Figure 1 (SEQ ID NO: 1), and the TSHβ subunit has at least one amino acid substitution selected from among positions 58-69 of the amino acid sequence of human TSHβ subunit as depicted in Figure 2 (SEQ ID NO: 2).
Paragraph 35. The TSH analog of Paragraph 30 in which the TSH β subunit is joined via a peptide bond at the carboxyl terminus to the amino terminus of the carboxyl terminal extension peptide of human chorionic gonadotropin, and in which the carboxyl terminus of the carboxyl terminal extension peptide is joined via a peptide bond to the amino terminus of α subunit.
Paragraph 36. The TSH analog of Paragraph 30 in which the TSHβ subunit is joined via a peptide bond at the carboxyl terminus to the amino terminus of the α subunit.
Paragraph 37. The mutant TSH heterodimer of Paragraph 24 wherein the hormonal half life in circulation *in vivo* of the mutant TSH heterodimer is greater than the wild type TSH.
Paragraph 38. The mutant TSH analog of Paragraph 30 wherein the hormonal half life in circulation *in vivo* of the mutant TSH analog is greater than the wild type TSH.
Paragraph 39, A nucleic acid comprising a nucleotide sequence encoding the mutant α subunit of Paragraph 1.
Paragraph 40. A nucleic acid comprising a nucleotide sequence encoding the TSH analog of Paragraph 30, in which the α subunit is joined to the β subunit via a peptide bond.
Paragraph 41. A method of treating or preventing hypothyroidism comprising administering to a subject in which such treatment or prevention is desired an amount of the TSH heterodimer of Paragraph 24 sufficient to treat or prevent hypothyroidism.
Paragraph 42. A method of treating or preventing hypothyroidism comprising administering to a subject in which such treatment or prevention is desired an amount of the TSH analog of Paragraph 30 sufficient to treat or prevent hypothyroidism.
Paragraph 43. A method of treating thyroid cancer comprising administering to a subject in which such treatment or prevention is desired an amount of the mutant TSH heterodimer of Paragraph 24 sufficient to stimulate iodine uptake and subsequently administering to said subject an amount of radiolabelled iodine sufficient to treat thyroid cancer.
Paragraph 44. A method of treating thyroid cancer comprising administering to a subject in which such treatment or prevention is desired an amount of the TSH analog of Paragraph 30 sufficient to stimulate iodine uptake and subsequently administering to said subject an amount of radiolabelled iodine sufficient to treat thyroid cancer.
Paragraph 45. A method of diagnosing thyroid cancer comprising administering to a subject an amount of the mutant TSH heterodimer of Paragraph 24 sufficient to stimulate uptake of iodine by thyroid cancer cells and an amount of radiolabelled iodine sufficient to diagnose thyroid cancer; and detecting said radiolabelled iodine, wherein an increase relative to a subject not having thyroid disease in uptake of radiolabelled iodine indicates that the subject has thyroid cancer.
Paragraph 46. A method of diagnosing thyroid cancer comprising administering to a subject lacking non-cancerous thyroid cells an amount of the mutant TSH heterodimer of Paragraph 24 sufficient to stimulate uptake of iodine by thyroid cancer cells and an amount of radiolabelled iodine sufficient to diagnose thyroid cancer; and detecting said radiolabelled iodine, wherein an increase in uptake of radiolabelled iodine indicates that the subject has thyroid cancer.
Paragraph 47. A method of diagnosing thyroid cancer comprising administering to a first subject an amount of the mutant TSH heterodimer of Paragraph 24 sufficient to stimulate release of thyroglobulin *in vivo* and measuring the levels of thyroglobulin in said first subject, in which an increase in thyroglobulin levels relative to the thyroglobulin levels in a sample of a second subject not having thyroid cancer indicates that said first subject has thyroid cancer.
Paragraph 48. A method of diagnosing thyroid cancer comprising administering to a subject an amount of the TSH analog of Paragraph 30 sufficient to stimulate uptake of iodine by thyroid cancer cells and an amount of radiolabelled iodine sufficient to diagnose thyroid cancer; and detecting said radiolabelled iodine, wherein an increase relative to a subject not having thyroid disease in uptake of radiolabelled iodine indicates that the subject has thyroid cancer.
Paragraph 49. A method of diagnosing thyroid cancer comprising administering to a subject lacking non-cancerous thyroid cells an amount of the mutant TSH heterodimer of Paragraph 30 sufficient to stimulate uptake of iodine by thyroid cancer cells and an amount of radiolabelled iodine sufficient to diagnose thyroid cancer; and detecting said radiolabelled iodine, wherein an increase uptake of radiolabelled iodine indicates that the subject has thyroid cancer.
Paragraph 50. A method of diagnosing thyroid cancer comprising administering to a first subject an amount of the TSH analog of Paragraph 30 sufficient to stimulate release of thyroglobulin *in vivo* and measuring the levels of thyroglobulin in said first subject, in which an increase in thyroglobulin levels relative to the thyroglobulin levels in a sample of a second subject not having thyroid cancer indicates that said first subject has thyroid cancer.
Paragraph 51. A method of diagnosing or screening for a disease or disorder characterized by the presence of antibodies against the TSH receptor comprising contacting cultured cells or isolated membrane containing TSH receptors with a sample putatively containing antibodies from a first subject and with a diagnostically effective amount of the radiolabelled mutant TSH heterodimer of Paragraph 24; and measuring the binding of the radiolabelled mutant TSH to the cultured cells or isolated membrane, wherein a decrease in the binding of the radiolabelled TSH relative to the binding in the absence of said sample or in the presence of a sample of a second subject not having said disease or disorder, indicates the presence of said disease or disorder in said first subject.
Paragraph 52. The method of Paragraph 51 in which said disease or disorder is Graves' disease.
Paragraph 53. A method of diagnosing or screening for a disease or disorder characterized by the presence of antibodies against the TSH receptor comprising contacting cultured cells or isolated membrane containing TSH receptors with a sample putatively containing antibodies from a first subject and with a diagnostically effective amount of the radiolabelled TSH analog of Paragraph 30; and measuring the binding of the radiolabelled mutant TSH to the cultured cells or isolated membrane, wherein a decrease in the binding of the radiolabelled TSH relative to the binding in the absence of said sample or in the presence of a sample of a second subject not having said disease or disorder, indicates the presence of said disease or disorder in said first subject.
Paragraph 54. The method of Paragraph 53 in which said disease or disorder is Graves' disease.
Paragraph 55. A pharmaceutical composition comprising a therapeutically effective amount of the mutant TSH heterodimer of Paragraph 12; and a pharmaceutically acceptable carrier.
Paragraph 56. A pharmaceutical composition comprising a therapeutically effective amount of the mutant TSH heterodimer of Paragraph 24; and a pharmaceutically acceptable carrier.
Paragraph 57. A pharmaceutical composition comprising a therapeutically effective amount of the TSH analog of Paragraph 30; and a pharmaceutically acceptable carrier.
Paragraph 58. A diagnostic composition comprising an amount of the mutant TSH heterodimer of Paragraph 12 sufficient to stimulate iodine uptake by thyroid cancer cells; and a pharmaceutically acceptable carrier.
Paragraph 59. A diagnostic composition comprising an amount of the mutant TSH heterodimer of Paragraph 24 sufficient to stimulate iodine uptake by thyroid cancer cells; and a pharmaceutically acceptable carrier.
Paragraph 60. A diagnostic composition comprising an amount of the TSH analog of Paragraph 30 sufficient to stimulate iodine uptake by thyroid cancer cells; and a pharmaceutically acceptable carrier.
Paragraph 61. A kit comprising in one or more containers a therapeutically effective amount of the mutant TSH heterodimer of Paragraph 12, 24 or the TSH analog of Paragraph 30.
Paragraph 62. A kit comprising in one or more containers a diagnostically effective amount of the mutant TSH heterodimer of Paragraph 12 or 24 or the TSH analog of Paragraph 30.
Paragraph 63. The nucleic acid of Paragraph 38 or 40 which is isolated.
Paragraph 64. The composition of Paragraph 55, 56, 58 or 59 in which the mutant TSH heterodimer is purified.

Specifically, in relation to a TSH analogue as described, the α subunit may comprise an amino acid substitution to a positively charged or basic residue from lysine, arginine and histidine at position 22 of the amino acid sequence of the α subunit as depicted in Figure 1 (SEQ ID NO: 1). The TSH analogue may further comprise one or more amino acid substitutions at positions 11, 13, 14, 16, 17 or 20 of the amino acid sequence of the α subunit as depicted in Figure 1 (SEQ ID NO: 1). The TSH analogue may further comprise one or more amino acid substitutions in the β hairpin L1 loop of the α subunit (residues 8 to 30 as depicted in Figure 1 (SEQ ID NO: 1)). The one or more substitutions may be amino acids selected from the group consisting of arginine and lysine. The one or more substitutions may be selected from the group consisting of αT11K, αQ13K, αE14K, αP16K, αF17R, and αQ20K. The TSH analogue may be such that the only mutation is an amino acid substitution to arginine at position 22 of the amino acid sequence of the α subunit as depicted in Figure 1 (SEQ ID NO: 1).

## Claims

1. A TSH analogue comprising an α subunit which is covalently bound to a TSHβ subunit, in which at least one of the subunits comprises at least one amino acid substitution in its amino acid sequence, and in which the bioactivity of the TSH analogue is greater than the bioactivity of a TSH analogue comprising a wild-type α subunit covalently bound to a wild-type TSH β subunit.

2. A TSH analogue according to Claim 1, in which the at least one amino acid substitution is in amino acid residues selected from among positions 11-22 of the amino acid sequence of α subunit as depicted in Figure 1 (SEQ ID NO: 1).

3. A TSH analogue according to Claim 1 or 2, in which the at least one amino acid substitution is in amino acid residues selected from among positions 58-69 of the amino acid sequence of human TSH β subunit as depicted in Figure 2 (SEQ ID NO: 2).

4. A TSH analogue according to Claim 1, 2 or 3, in which both the α subunit and the β TSH subunit comprise one or more amino acid substitutions.

5. A TSH analogue according to any preceding claim, in which the α subunit has at least one amino acid substitution in amino acid residues selected from among positions 11-22 of the amino acid sequence of human α subunit as depicted in Figure 1 (SEQ ID NO: 1), and the TSH β subunit has at least one amino acid substitution selected from among positions 58-69 of the amino acid sequence of human TSH β subunit as depicted in Figure 2 (SEQ ID NO: 2).

6. A TSH analogue according to any preceding claim, in which the TSH β subunit is joined via a peptide bond at the carboxyl terminus to the amino terminus of the carboxyl terminal extension peptide of human chorionic gonadotropin, and in which the carboxyl terminus of the carboxyl terminal extension peptide is joined via a peptide bond to the amino terminus of α subunit.

7. A TSH analogue according to any preceding claim, in which the TSH β subunit is joined via a peptide bond at the carboxyl terminus to the amino terminus of the α subunit.

8. A TSH analogue according to any preceding claim, in which the hormonal half life in circulation *in vivo* of the mutant TSH analogue is greater than the wild type TSH.

9. A nucleic acid comprising a nucleotide sequence encoding a TSH analogue according to any preceding claim, in which the α subunit is joined to the β subunit via a peptide bond.

10. A TSH analogue according to any of Claims 1 to 8 for use in a method of treating or preventing hypothyroidism, the method comprising administering to a subject in which such treatment or prevention is desired an amount of a TSH analogue according to any of Claims 1 to 8, sufficient to treat or prevent hypothyroidism.

11. A TSH analogue according to any of Claims 1 to 8 for use in a method of treating thyroid cancer, the method comprising administering to a subject in which such treatment or prevention is desired an amount of a TSH analogue according to any of Claims 1 to 8 sufficient to stimulate iodine uptake and subsequently administering to the subject an amount of radiolabelled iodine sufficient to treat thyroid cancer.

12. A TSH analogue according to any of Claims 1 to 8 for use in a method of diagnosing thyroid cancer, the method comprising administering to a subject an amount of a TSH analogue according to any of Claims 1 to 8 sufficient to stimulate uptake of iodine by thyroid cancer cells and an amount of radiolabelled iodine sufficient to diagnose thyroid cancer; and detecting the radiolabelled iodine, in which an increase relative to a subject not having thyroid disease in uptake of radiolabelled iodine indicates that the subject has thyroid cancer.

13. A TSH analogue according to any of Claims 1 to 8 for use in a method of diagnosing thyroid cancer, the method comprising administering to a subject lacking non-cancerous thyroid cells an amount of a TSH analogue according to any of Claims 1 to 8 sufficient to stimulate uptake of iodine by thyroid cancer cells and an amount of radiolabelled iodine sufficient to diagnose thyroid cancer; and detecting the radiolabelled iodine, in which an increase uptake of radiolabelled iodine indicates that the subject has thyroid cancer.

14. A TSH analogue according to any of Claims 1 to 8 for use in a method of diagnosing thyroid cancer, the method comprising administering to a first subject an amount of a TSH analogue according to any of Claims 1 to 8 sufficient to stimulate release of thyroglobulin *in vivo* and measuring the levels of thyroglobulin in the first subject, in which an increase in thyroglobulin levels relative to the thyroglobulin levels in a sample of a second subject not having thyroid cancer indicates that the first subject has thyroid cancer.

15. A method of diagnosing or screening for a disease or disorder **characterized by** the presence of antibodies against a TSH receptor comprising contacting cultured cells or isolated membrane containing TSH receptors with a sample putatively containing antibodies from a first subject and with a diagnostically effective amount of a radiolabelled TSH analogue according to any of Claims 1 to 8; and measuring the binding of the radiolabelled mutant TSH to the cultured cells or isolated membrane, in which a decrease in the binding of the radiolabelled TSH relative to the binding in the absence of the sample or in the presence of a sample of a second subject not having the disease or disorder, indicates the presence of the disease or disorder in the first subject.

16. A method according to Claim 15 in which the disease or disorder is Graves' disease.

17. A pharmaceutical composition comprising a therapeutically effective amount of a TSH analogue according to any of Claims 1 to 8; and a pharmaceutically acceptable carrier.

18. A diagnostic composition comprising an amount of a TSH analogue according to any of Claims 1 to 8 sufficient to stimulate iodine uptake by thyroid cancer cells; and a pharmaceutically acceptable carrier.

19. A kit comprising in one or more containers a therapeutically effective amount of a TSH analogue according to any of Claims 1 to 8.

20. A kit comprising in one or more containers a diagnostically effective amount of the a TSH analogue according to any of Claims 1 to 8.


### Annex to the application documents - subsequently filed sequences listing

SEQUENCE LISTING

<110> University of Maryland, Baltimore

<120> MUTANTS OF THYROID STIMULATING HORMONE
  AND METHODS BASED THEREON

<130> UOFMD.002QPC

<140> PCT US98/19772
<141> 1998-09-22

<150> 08/939,472
<151> 1997-09-22

<160> 4

<170> FastSEQ for Windows Version 3.0

<210> 1
<211> 91
<212> PRT
<213> Homo sapiens

<400> 1
Ala Pro Asp Val Gln Asp Cys Pro Glu Cys Thr Leu Gln Glu Asn Pro
 1               5                  10                  15
Phe Phe Ser Gln Pro Gly Ala Pro Ile Leu Gln Cys Met Gly Cys Cys
            20                  25                  30
Phe Ser Arg Ala Tyr Pro Thr Pro Leu Arg Ser Lys Lys Thr Met Leu
        35                  40                  45
Val Gln Lys Asn Val Ser Glu Ser Thr Cys Cys Val Ala Lys Ser Tyr
    50                  55                  60
Asn Arg Val Thr Val Met Gly Gly Phe Lys Val Glu Asn His Thr Ala
65                  70                  75                  80
Cys His Cys Ser Thr Cys Tyr Tyr His Lys Ser
                85                  90

<210> 2
<211> 118
<212> PRT
<213> Homo sapien

<400> 2
Phe Cys Ile Pro Thr Glu Tyr Thr Met His Ile Glu Arg Arg Glu Cys
 1               5                  10                  15
Ala Tyr Cys Leu Thr Ile Asn Thr Thr Ile Cys Ala Gly Tyr Cys Met
            20                  25                  30
Thr Arg Asp Ile Asn Gly Lys Leu Phe Leu Pro Lys Tyr Ala Leu Ser
        35                  40                  45
Gln Asp Val Cys Thr Tyr Arg Asp Phe Ile Tyr Arg Thr Val Glu Ile
    50                  55                  60
Pro Gly Cys Pro Leu His Val Ala Pro Tyr Phe Ser Tyr Pro Val Ala
65                  70                  75                  80
Leu Ser Cys Lys Cys Gly Lys Cys Asn Thr Asp Tyr Ser Asp Cys Ile
                85                  90                  95
His Glu Ala Ile Lys Thr Asn Tyr Cys Thr Lys Pro Gln Lys Ser Tyr
            100                 105                 110
Leu Val Gly Phe Ser Val

        115

<210> 3
<211> 530
<212> DNA
<213> Homo sapien

<220>
<221> CDS
<222> (86)...(520)

<400> 3
agacaaggca ggggacgcac caaggatgga gatgttccag gggctgctgc tgttgctgct 60
gctgagcatg ggcgggacat gggca tcc aag gag ccg ctt cgg cca cgg tgc 112
Ser Lys Glu Pro Leu Arg Pro Arg Cys
1               5

cgc ccc atc aat gcc acc ctg gct gtg gag aag gag ggc tgc ccc gtg 160
Arg Pro Ile Asn Ala Thr Leu Ala Val Glu Lys Glu Gly Cys Pro Val
10                  15                  20                  25

tgc atc acc gtc aac acc acc atc tgt gcc ggc tac tgc ccc acc atg 208
Cys Ile Thr Val Asn Thr Thr Ile Cys Ala Gly Tyr Cys Pro Thr Met
                30                  35                  40

acc cgc gtg ctg cag ggg gtc ctg ccg gcc ctg cct cag gtg gtg tgc 256
Thr Arg Val Leu Gln Gly Val Leu Pro Ala Leu Pro Gln Val Val Cys
            45                  50                  55

aac tac cgc gat gtg cgc ttc gag tcc atc cgg ctc cct ggc tgc ccg 304
Asn Tyr Arg Asp Val Arg Phe Glu Ser Ile Arg Leu Pro Gly Cys Pro
        60                  65                  70

cgc ggc gtg aac ccc gtg gtc tcc tac gcc gtg gct ctc agc tgt caa 352
Arg Gly Val Asn Pro Val Val Ser Tyr Ala Val Ala Leu Ser Cys Gln
    75                  80                  85

tgt gca ctc tgc cgc cgc agc acc act gac tgc ggg ggt ccc aag gac 400
Cys Ala Leu Cys Arg Arg Ser Thr Thr Asp Cys Gly Gly Pro Lys Asp
90                  95                  100                 105

cac ccc ttg acc tgt gat gac ccc cgc ttc cag gac tcc tct tcc tca 448
His Pro Leu Thr Cys Asp Asp Pro Arg Phe Gln Asp Ser Ser Ser Ser
                110                 115                 120

aag gcc cct ccc ccc agc ctt cca agc cca tcc cga ctc ccg ggg ccc 496
Lys Ala Pro Pro Pro Ser Leu Pro Ser Pro Ser Arg Leu Pro Gly Pro
            125                 130                 135

tcg gac acc ccg atc ctc cca caa taaaggcttc 530
Ser Asp Thr Pro Ile Leu Pro Gln
        140                 145


<210> 4
<211> 9
<212> DNA
<213> Homo sapien

<400> 4
tcaatccgc 9

-3-
-3-